# EUROPEAN PATENT APPLICATION

(11) **EP 4 299 124 A1**
(43) Date of publication of application: **03.01.2024**
(21) Application number: 22305966.8
(22) Date of filing: 30.06.2022
(51) Int. Cl.: A61P 25/00, A61K 51/10, C07K 16/28, A61K 39/395

(54) **ANTI-MGLUR2 NANOBODIES FOR USE AS BIOMOLECULE TRANSPORTER**

(71) Applicant: UNIVERSITE DE MONTPELLIER, 34090 Montpellier (FR); Centre National de la Recherche Scientifique (CNRS), 75016 Paris (FR); Institut National de la Santé et de la Recherche Médicale (INSERM), 75013 Paris (FR)
(72) Inventor: PIN, Jean-Philippe, 34190 CAZILHAC (FR); RONDARD, Philippe, 34980 SAINT-GÉLY-DU-FESC (FR); PREZEAU, Laurent, 34730 PRADES-LE-LEZ (FR); KNIAZEFF, Julie, 34730 PRADES-LE-LEZ (FR); OOSTERLAKEN, Mathieu, 34090 MONTPELLIER (FR); TSITOKANA, Mireille Elodie, 34080 MONTPELLIER (FR)
(74) Representative: Regimbeau

(57) **Abstract**

The present invention relates to a polypeptide comprising at least one single domain antibody directed against mGluR2 for use as a biomolecule transporter crossing the blood-brain barrier (BBB), with the proviso that the polypeptide does not comprise another binding domain directed against a receptor on the vascular endothelium of the blood-brain barrier or a Fc portion of an immunoglobulin binding domain. In particular, the said polypeptide is used for transporting detectable label or therapeutic agent.

## Description

### TECHNICAL FIELD OF THE INVENTION

The present invention relates to new use of anti-mGluR2 nanobodies as biomolecule transporter crossing the blood-brain barrier (BBB).

### BACKGROUND ART

Most brain diseases are still in need of new drugs or new therapeutic ways.

To date, mostly small molecules are used and developed for these pathologies, but they sometimes lack selectivity or show off-target binding resulting in side effects. Immunotherapies have shown to be efficient in many medical areas, but there is no antibody-based therapeutics for a brain disease to date, mostly due to their little brain penetration.

Actually, the 2 main ways for biological tools to target the brain are:
- A physical approach using ultra-sounds opening the BBB (Choi et al., 2018; Wu et al. 2017), but this approach is invasive and difficult to manage for repeted treatments;
- The (main) use of transcytose mechanism and transferrin or insulin receptors to cross the BBB, such as anti-transferrin antibodies and the transcytose mechanism of this transferrin receptor to help therapeutic agents to cross endothelial cells constituting the blood-brain barrier (Bickel et al., 2001).

But there is still a need to develop new ways to target the brain.

The Applicant observed that injection of some anti-mGluR2 nanobodies directly in the brain or *via* intraperitoneal administration, improve behaviorial deficits in a mouse model of psychosis. This indicated that anti-mGluR2 nanobodies were able to penetrate in sufficient amount in the brain to act in central nervous system. Another surprising observation was that the effects of the anti-mGluR2 nanobodies injected by intraperitoneal route still remain after 7 days after a sole injection, whereas the nanobodies, which are small molecules, are usually eliminated from circulation by the renal filter. This demonstrates that the anti-mGluR2 nanobodies are able to cross the blood-brain barrier (BBB), at least during the 12 hours following the injection, and stay in the brain for a longer period, indicating that the anti-mGluR2 nanobodies cross the BBB in a directional way (coming in easily but coming out less quickly).

So, the Applicant surprisingly demonstrated that some anti-mGluR2 nanobodies were able to cross the blood-brain barrier (BBB), opening a new therapeutic pathway using such nanobodies as biomolecule transporter. The anti-mGluR2 nanobodies of the present invention may transport a detectable label for use in biological and/or diagnostic analysis, or a therapeutic agent for use in the treatment of a neurological or psychiatric disorder in a subject in need thereof.

This approach is advantageous as it permits repeted uses contrary to the physical process using ultra-sounds and the monodirectional crossing improves the biodisponibility of the detectable labeled and therapeutic agents.

### SUMMARY OF THE INVENTION

The present invention relates to an isolated single domain antibody directed against mGluR2 or a polypeptide comprising at least one single domain antibody directed against mGluR2 for use as a biomolecule transporter crossing the blood-brain barrier (BBB).

In particular, when the biomolecule transporter according to the invention only comprises a single domain antibody directed against mGluR2, it does not comprise another binding domain directed against a receptor on the vascular endothelium of the blood-brain barrier or a Fc portion of an immunoglobulin binding domain.

In a particular embodiment, the biomolecule is a detectable label.

In another embodiment, the biomolecule is a therapeutic agent for the treatment of a neurological or psychiatric disorder.

The term "biomolecule" in the present invention means a biological molecule, and encompassess a peptide, a polypeptide or a protein, including an antibody.

### DESCRIPTION OF THE FIGURES

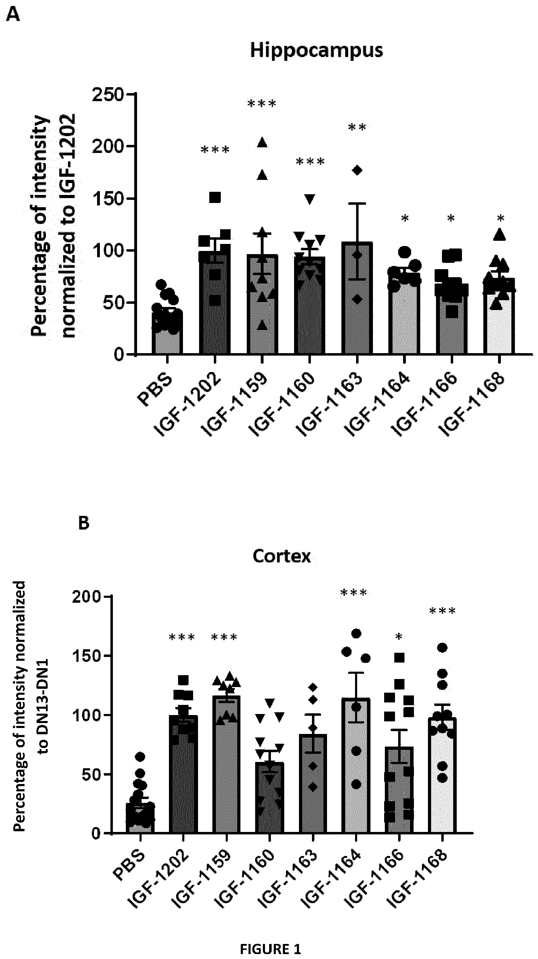

**Figure 1****:** mGu2-targeting nanobodies can cross the blood-brain barrier. Specific labelling of mGlu2-targeting nanobodies (IGF-1202, IGF-1159, IGF-1160, IGF-1163, IGF-1164, IGF-1166 and IGF-1168) in the hippocampus (A) and the cortex (B), revealed by immunohistochemistry on sagittal slices from mice 24 h after intraperitoneal administration (10 mg/kg). Background labelling in PBS administred control mouse is also illustrated. Quantitative data are shown as means ± SEM. One-way ANOVA - Holm-sidak *post-hoc* test (A) and non-parametric Kruskal-Wallis - Dunn's *post-hoc* test (B) were used to compare more than two groups (comparison of all conditions to PBS ). All values for which p<0.05 were considered significant (*p<0.05, **p<0.01, ***p<0.001).

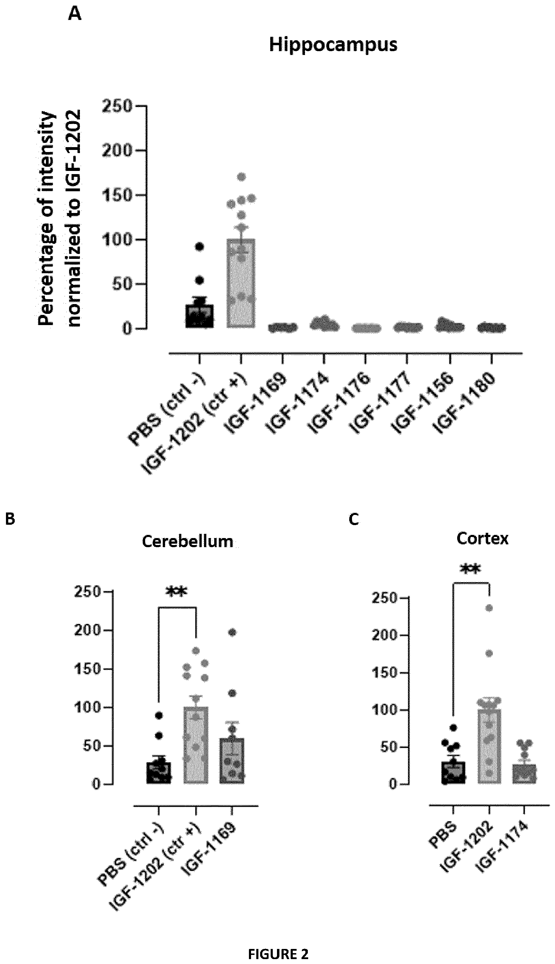

**Figure 2****:** Nanobodies targeting mGluRs different from mGlu2 cannot be detected in the brain. Specific labelling of nanobodies targeting mGlu2 (IGF-1202), mGlu4 (IGF-1169), mGlu5 (IGF-1174, IGF-1176, IGF-1177, IGF-1156) and mGlu7 (IGF-1180) in the hippocampus (A), the cerebellum (B), and the cortex (C), revealed by immunohistochemistry on sagittal slices from mice 25-31 hours after intraperitoneal administration (10 mg/kg). Background labelling in PBS administred control mouse is also illustrated. Quantitative data are shown as means ± SEM. Non-parametric Kruskal-Wallis - Dunn's *post-hoc* test were used to compare more than two groups (comparison of all conditions to PBS ). All values for which p<0.05 were considered significant (**p<0.01).

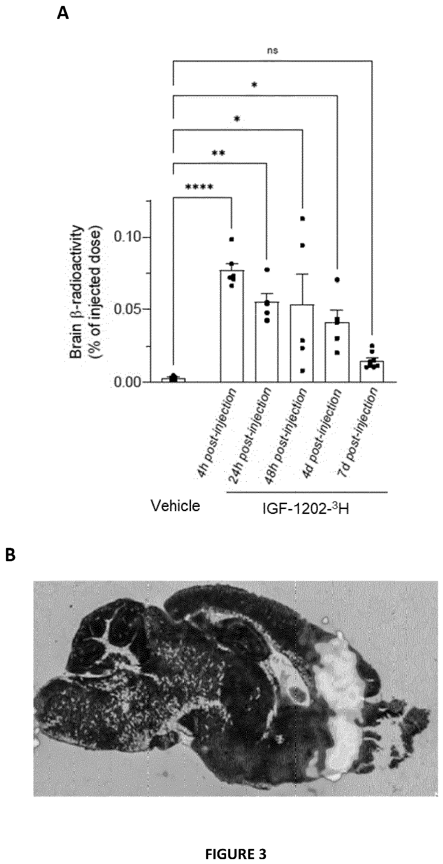

**Figure 3****:** IGF-1202 remains several days in the brain parenchyma. (A) Quantification of radioactivity present in the mouse brain 4h, 24h, 48h, 4 days or 7 days after i.p. administration of [³H]-IGF-1202 at 10 mg/kg (3 pCi/mice). Quantitative data are shown as means ± SEM. Non-parametric Kruskal-Wallis test was used to compare more than two groups. All values for which p<0.05 were considered significant (*p<0.05, **p<0.01, ***p<0.001, ****p<0.0001. (B) Autoradiographic signal in mouse brain 24 h after i.p. administration of [³H]-IGF-1202 .

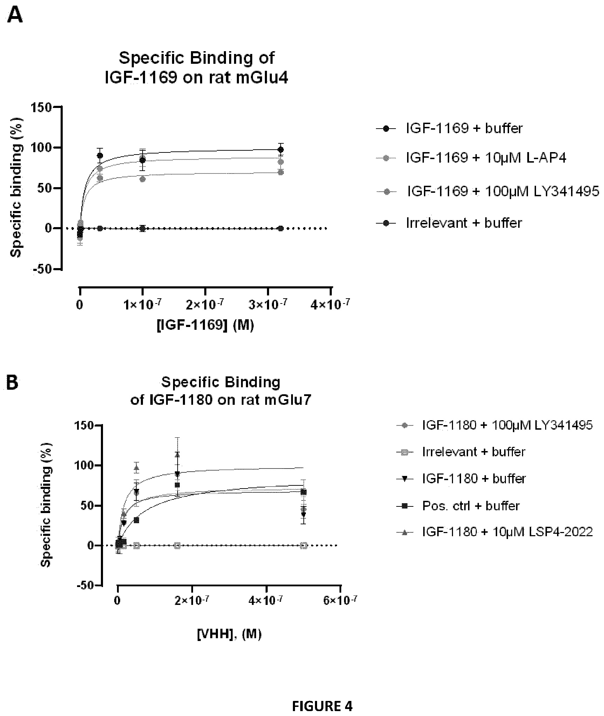

**Figure 4****:** Representative binding affinities of the nanobodies tested *in vivo.* (A) In the presence of L-AP4 (agonist), LY 341495 (antagonist) or buffer, IGF-1169 shows an affinity of 8.4 nM, 7.8 nM and 7.4 nM for the rat mGlu4, respectively. (B) In the presence of LSP4-2022 (agonist), LY 341495 (antagonist) or buffer, IGF-1180 shows an affinity of 16 nM, 19 nM and 15 nM for the rat mGlu7, respectively.

### DETAILED DESCRIPTION OF THE INVENTION

The present invention relates to an isolated single domain antibody directed against mGluR2 or a polypeptide comprising at least one single domain antibody directed against mGluR2 for use as a biomolecule transporter crossing the blood-brain barrier (BBB).

In particular, when the biomolecule transporter according to the invention only comprises a single domain antibody directed against mGluR2, it does not comprise another binding domain directed against a receptor on the vascular endothelium of the blood-brain barrier or a Fc portion of an immunoglobulin binding domain.

The sequences illustrated below are listed in the following table 1:

**Table 1**

| **SEQ ID NO :** | **SEQUENCE** | **DESCRIPTION** |
|---|---|---|
| 1 | GRTFSSFA | CDR1 sequence of IGF-1159 nanobody |
| 2 | ISWSGST | CDR2 sequence of IGF-1159 nanobody |
| 3 | ALGKGSSYYYSPSGYDY | CDR3 sequence of IGF-1159 nanobody |
| 4 | GRTFSTYG | CDR1 sequence of IGF-1160 nanobody |
| 5 | INWRDDVT | CDR2 sequence of IGF-1160 nanobody |
| 6 | AAQGRGSFKSIFERSRYDS | CDR3 sequence of IGF-1160 nanobody |
| 7 | G RTFSDLA | CDR1 sequence of IGF-1164 nanobody |
| 8 | INWSGHSI | CDR2 sequence of IGF-1164 nanobody |
| 9 | AADTTGGTYKLRTKEYDY | CDR3 sequence of IGF-1164 nanobody |
| 10 | GRTFRPYG | CDR1 sequence of IGF-1163 nanobody |
| 11 | IIWSLGYT | CDR2 sequence of IGF-1163 nanobody |
| 12 | AARDRSSSEYDY | CDR3 sequence of IGF-1163 nanobody |
| 13 | GRTFSIYS | CDR1 sequence of IGF-1166 nanobody |
| 14 | ITWRREYT | CDR2 sequence of IGF-1166 nanobody |
| 15 | ALRPGLRDDLNY | CDR3 sequence of IGF-1166 nanobody |
| 16 | VRFFSINT | CDR1 sequence of IGF-1168 nanobody |
| 17 | ITSSGST | CDR2 sequence of IGF-1168 nanobody |
| 18 | HADYKYTTHNTA | CDR3 sequence of IGF-1168 nanobody |
| 19 | QVQLVQSGGGLVQAGGSLRLSCAASGRTFSSFAM AWFRQAPGKEREFVAAISWSGSTGHADSVKGRFT ITRDNAKNTVFLQMNSLKPEDTAVYYCALGKGSSY YYSPSGYDYWGQGTQVTVSS | amino acid sequence of IGF-1159 nanobody |
| 20 | EVQLVESGGGLVQAGDSLRLSCAASGRTFSTYGM AWFRQAPGKEREFVARINWRDDVTYYADSLKGRF TISRDKAKNTVYLQLDSLTPEDTAVYYCAAQGRGS FKSIFERSRYDSWGQGTQVTVSS | amino acid sequence of IGF-1160 nanobody |
| 21 | EVQLVESGGGLVQAGGSLRLSCAASGRTFSDLAM GWFRQAPGKEREFVAAINWSGHSIYYTDSVKARF TISRDNAKDTVYLQMNSLKSEDTAVYFCAADTTG GTYKLRTKEYDYRGQGTQVTVSS | amino acid sequence of IGF-1164 nanobody |
| 22 | QVQLVQSGGGYVQAGGSLSVSCAASGRTFRPYG VGWFRQAPGKEREFVAAIIWSLGYTIYADSVKGRF TISRDNAKNTVYLQMNSLKAEDTAVYYCAARDRSS SEYDYWGQGTQVTVSS | amino acid sequence of IGF-1163 nanobody |
| 23 | EVQLVESGGGVVQPGDSLRLSCVASGRTDSIYSMA WFRQAPGKEREFVAIITWRREYTNYEDSVRGRFTI SRDNAKNAVYLQMNKLKPEDTAVYYCALRPGLRD DLNYWGQGTQVTVSS | amino acid sequence of IGF-1166 nanobody |
| 24 | QVQLVQSGGGLVQAGGSLRLSCAASVRFFSINTMG WYRQAPGKQRELVADITSSGSTNYADSGKGRFTIS RDNAKNTVYLQMNRLKPEDTAVYYCHADYKYTTH NTAWGQGTQVTVSS | amino acid sequence of IGF-1168 nanobody |
| 25 | EPKIPQPQPKPQPQPQPQPQPKPQPKPEP | linker sequence L1 |
| 26 | GGGGS GGGGS GGGGS | linker sequence L2 |
| 27 | GGGGS GGGGS GGGGS GGGGS | linker sequence L3 |
| 28 | GGGGS | linker sequence L4 |
| 29 | GGS | linker sequence L5 |
| 30 | GGG | linker sequence L6 |
| 31 | GGGSS GGGSS GGGSS | linker sequence L7 |
| 32 | QVQLVQSGGGLVQAGGSLRLSCAASVRFFSINTMG WYRQAPGKQRELVADITSSGSTNYADSGKGRFTIS RDNAKNTVYLQMNRLKPEDTAVYYCHADYKYTTH NTAWGQGTQVTVSSEPKIPQPQPKPQPQPQPQPQ PKPQPKPEPQVQLVQSGGGLVQAGGSLRLSCAASG RTFSSFAMAWFRQAPGKEREFVAAISWSGSTGHAD SVKGRFTITRDNAKNTVFLQMNSLKPEDTAVYYCAL GKGSSYYYSPSGYDYWGQGTQVTVSS | Amino acid sequence of the biparatopic IGF1202 (construct IGF1168-linker sequenceL1-IGF1159) |
| 33 | QVQLVQSGGGLVQAGGSLRLSCAASVRFFSINTMG WYRQAPGKQRELVADITSSGSTNYADSGKGRFTIS RDNAKNTVYLQMNRLKPEDTAVYYCHADYKYTTH NTAWGQGTQVTVSSEPKIPQPQPKPQPQPQPQPQ PKPQPKPEPQVQLVQSGGGLVQAGGSLRLSCAASG RTFSSFAMAWFRQAPGKEREFVAAISWSGSTGHAD SVKGRFTITRDNAKNTVFLQMNSLKPEDTAVYYCAL GKGSSYYYSPSGYDYWGQGTQVTVSSLEHHHHHH | Amino acid sequence of the biparatopic IGF1202-tag6xHIS |
| 34 | caggtgcagctggtgcagtctgggggaggattggtgcaggct gggggctctctgagactctcctgtgcagcctctggacgcacc tttagtagcttcgccatggcctggttccgccaggctccaggg aaggagcgtgagtttgtagcagctattagctggagtggtagt acaggccatgcagactctgtgaagggccgattcaccatcac cagagacaacgccaagaacacggtgtttctgcaaatgaaca gcctgaaacctgaggacacggccgtttattactgtgcactag gaaaggggagtagttactactacagtcctagtgggtatgact | nucleic acid sequence of IGF-1159 nanobody |
| | actgggggccaggggacccaggtcaccgtctcctca | |
| 35 | gaggtgcagctggtggagtctggtggaggattggtgcaggct ggggactctctgagactctcctgtgcagcctctggacgcacc tttagtacctatggcatggcctggttccgccaggctccgggg aaggagcgtgagtttgtggcgcgaattaactggcgcgatgat gtcacatactatgcagactccctgaagggccgattcaccatc tcccgagacaaagccaagaacacggtgtatctgcaactgga cagcctgactcctgaggacacggccgtttattactgtgcagc ccaaggtcgcggtagttttaaatcaatcttcgagcggtcgcg ctatgactcctggggccaggggacccaggtcaccgtctcct ca | nucleic acid sequence of IGF-1160 nanobody |
| 36 | gaggtgcagctggtggagtctgggggaggattggtgcaggct gggggctctctgagactgtcctgtgcagcctctggacgcacc ttcagtgaccttgccatgggctggttccgccaggctccagga aaggagcgtgagtttgtagcagccattaactggagtggtcat agcatatactatacagactccgtgaaggcccgattcaccatc tccagagacaacgccaaagacacggtgtatctgcaaatgaa cagcctgaaatctgaggacacggccgtttatttttgtgcagc agatacgactggtggcacctataaactccggacgaaggagt atgactaccgggggcaggggacccaggtcaccgtctcctca | nucleic acid sequence of IGF-1164 nanobody |
| 37 | caggtgcagctggtgcagtctgggggaggatatgtgcaggct gggggctctctgagcgtctcctgtgcagcctctggacgaacc ttccgtccctatggcgtgggctggttccgccaggctccaggg aaggagcgtgagtttgtagcagctattatttggagtcttggat acacaatttatgcagactccgtgaagggccgattcaccatct ccagagacaacgccaagaacacggtttatctgcaaatgaac agcctgaaagctgaggacacggccgtttattactgtgcagcc cgcgatcgtagttcgtcagagtat gactactggggccaggggacccaggtcaccgtctcctca | nucleic acid sequence of IGF-1163 nanobody |
| 38 | gaggtgcagctggtggagtctgggggaggagtggtgcagcct ggggattctctgagactctcttgtgtagcctctggacgcaccg atagtatttattccatggcctggttccgccaggctccagggaa ggaacgtgagtttgtagcgattattacctggagacgtgagtac acaaattatgaggactccgtgaggggccgattcaccatctcc agagacaacgccaagaacgcggtgtatctgcaaatgaacaa | nucleic acid sequence of IGF-1166 nanobody |
| | gttgaaacctgaggacacggccgtttattactgtgcacttagg ccaggcctacgcgatgacttaaactactggggccaggggacc caggtcaccgtctcctca | |
| 39 | caggtgcagctggtgcagtctgggggaggcttggtgcaggct ggggggtctctgagactctcctgtgcagcctctgtaaggttct tcagtattaataccatgggctggtaccgccaggctccaggga agcagcgcgagttggtcgcagatattactagtagtggtagca caaactatgcagactccgggaagggccgattcaccatctcc agagacaacgccaagaacacggtgtatctgcagatgaaca ggttgaaacctgaggacacagccgtctattactgccatgcgg attacaaatacacaacccataacacggcctggggccagggg acccaggtcaccgtctcctca | nucleic acid sequence of IGF-1168 nanobody |
| 40 | caggtgcagcttgtacagtctgggggaggcttggtgcaggct ggggggtctctgagactctcctgtgcagcctctgtaaggttct tcagtattaataccatgggctggtatcgccaggctccaggga agcagcgcgagttggtcgcagatattactagtagtggtagca caaactatgcagactccgggaagggccgattcaccatctcc agagacaacgccaagaacacggtgtatctgcagatgaaca ggttgaaacctgaggacacagccgtctattactgccatgcg gattacaaatacacaacccataacacggcctggggccagg gtacccaggtcaccgtctcctcagaaccgaagatcccgcag ccgcagccaaaacctcagccgcagccacagccgcaacctc aaccgaaaccgcaaccaaaaccggaacctcaggtgcaatt ggtgcagtctgggggaggattggtgcaggctgggggctctct gagactctcctgtgcagcctctggacgcacctttagtagctt cgccatggcctggttccgccaggctccagggaaggagcgtg agtttgtagcagctattagctggagtggtagtacaggccatg cagactctgtgaagggccgattcaccatcaccagagacaac gccaagaacacggtgtttctgcaaatgaacagcctgaaacc tgaggacacggccgtttattactgtgcactaggaaagggga gtagttactactacagtcctagtgggtatgactactggggcc aggggacccaggtcaccgtctcctca | nucleic acid sequence of the biparatopic IGF1202 (construct IGF1168-linker sequenceL1-IGF1159) |
| 41 | caggtgcagcttgtacagtctgggggaggcttggtgcaggct ggggggtctctgagactctcctgtgcagcctctgtaaggttct tcagtattaataccatgggctggtatcgccaggctccaggga | nucleic acid sequence of the biparatopic IGF1202-tag6xHIS |
| | agcagcgcgagttggtcgcagatattactagtagtggtagca caaactatgcagactccgggaagggccgattcaccatctcc agagacaacgccaagaacacggtgtatctgcagatgaaca ggttgaaacctgaggacacagccgtctattactgccatgcg gattacaaatacacaacccataacacggcctggggccagg gtacccaggtcaccgtctcctcagaaccgaagatcccgca gccgcagccaaaacctcagccgcagccacagccgcaacct caaccgaaaccgcaaccaaaaccggaacctcaggtgcaat tggtgcagtctgggggaggattggtgcaggctgggggctctc tgagactctcctgtgcagcctctggacgcacctttagtagctt cgccatggcctggttccgccaggctccagggaaggagcgtg agtttgtagcagctattagctggagtggtagtacaggccatg cagactctgtgaagggccgattcaccatcaccagagacaac gccaagaacacggtgtttctgcaaatgaacagcctgaaacc tgaggacacggccgtttattactgtgcactaggaaagggga gtagttactactacagtcctagtgggtatgactactggggcc aggggacccaggtcaccgtctcctcactcgagcaccaccac caccaccac | |
| 42 | QVQLQESGGGLVQPGGSLRLSCEASGFTFSRFGMT WVRQAPGKGVEWVSGISSLGDSTLYADSVKGRFTI SRDNAKNTLYLQMNSLKPEDTAVYYCTIGGSLNPG GQGTQVTVSS | Amino acid sequence of the nanobody against Albumine (Alb8) |
| 43 | EVQLQASGGGLVQAGGSLRLSCSASVRTFSIYAM GWFRQAPGKEREFVAGINRSGDVTKYADFVKGR FSISRDNAKNMVYLQMNSLKPEDTALYYCAATWA YDTVGALTSGYNFWGQGTQVTVSS | Amino acid sequence of the nanobody against Transferin receptor (Tfr) |
| 44 | QVQLVQSGGGLVQAGGSLRLSCAASVRFFSINTM GWYRQAPGKQRELVADITSSGSTNYADSGKGRF TISRDNAKNTVYLQMNRLKPEDTAVYYCHADYK YTTHNTAWGQGTQVTVSSGGGGSGGGGSGGG GSQVQLVQSGGGLVQAGGSLRLSCAASGRTFSS FAMAWFRQAPGKEREFVAAISWSGSTGHADSVK GRFTITRDNAKNTVFLQMNSLKPEDTAVYYCALG KGSSYYYSPSGYDYWGQGTQVTVSSGGGGSGG GGSEVQLQASGGGLVQAGGSLRLSCSASVRTFSI | Amino acid sequence of the biparatopic nanobody IGF-1267 (biparatopic IGF1201 with nanobody against Tfr in bold characters) |
| | YAMGWFRQAPGKEREFVAGINRSGDVTKYADFV KGRFSISRDNAKNMVYLQMNSLKPEDTALYYCAA TWAYDTVGALTSGYNFWGQGTQVTVSS | |
| 45 | QVQLVQSGGGLVQAGGSLRLSCAASVRFFSINTM GWYRQAPGKQRELVADITSSGSTNYADSGKGRF TISRDNAKNTVYLQMNRLKPEDTAVYYCHADYKY TTHNTAWGQGTQVTVSSEPKIPQPQPKPQPQPQ PQPQPKPQPKPEPQVQLVQSGGGLVQAGGSLRL SCAASGRTFSSFAMAWFRQAPGKEREFVAAISWS GSTGHADSVKGRFTITRDNAKNTVFLQMNSLKPE DTAVYYCALGKGSSYYYSPSGYDYWGQGTQVTV SSGGGGSGGGGSEVQLQASGGGLVQAGGSLRLS CSASVRTFSIYAMGWFRQAPGKEREFVAGINRSG DVTKYADFVKGRFSISRDNAKNMVYLQMNSLKPE DTALYYCAATWAYDTVGALTSGYNFWGQGTQV TVSS | Amino acid sequence of the biparatopic nanobody IGF-1268 (biparatopic IGF1202 with nanobody against Tfr in bold characters) |
| 46 | QVQLVQSGGGLVQAGGSLRLSCAASVRFFSINTM GWYRQAPGKQRELVADITSSGSTNYADSGKGRF TISRDNAKNTVYLQMNRLKPEDTAVYYCHADYK YTTHNTAWGQGTQVTVSSGGGGSGGGGSGGG GSQVQLVQSGGGLVQAGGSLRLSCAASGRTFSS FAMAWFRQAPGKEREFVAAISWSGSTGHADSV KGRFTITRDNAKNTVFLQMNSLKPEDTAVYYCA LGKGSSYYYSPSGYDYWGQGTQVTVSSGGGGS GGGGSQVQLQESGGGLVQPGGSLRLSCEASGF TFSRFGMTWVRQAPGKGVEWVSGISSLGDSTLY ADSVKGRFTISRDNAKNTLYLQMNSLKPEDTAVY YCTIGGSLNPGGQGTQVTVSS | Amino acid sequence of the biparatopic nanobody IGF-1269 (biparatopic IGF1201 with nanobody against Alb8 in bold characters) |
| 47 | QVQLVQSGGGLVQAGGSLRLSCAASVRFFSINTM GWYRQAPGKQRELVADITSSGSTNYADSGKGRF TISRDNAKNTVYLQMNRLKPEDTAVYYCHADYK YTTHNTAWGQGTQVTVSSEPKIPQPQPKPQPQ PQPQPQPKPQPKPEPQVQLVQSGGGLVQAGG SLRLSCAASGRTFSSFAMAWFRQAPGKEREFVA AISWSGSTGHADSVKGRFTITRDNAKNTVFLQM | Amino acid sequence of the biparatopic nanobody IGF-1270 (biparatopic IGF1202 with nanobody against Alb8 in bold characters) |
| | NSLKPEDTAVYYCALGKGSSYYYSPSGYDYWGQ GTQVTVSSGGGGSGGGGSQVQLQESGGGLVQ PGGSLRLSCEASGFTFSRFGMTWVRQAPGKGV EWVSGISSLGDSTLYADSVKGRFTISRDNAKNTL YLQMNSLKPEDTAVYYCTIGGSLNPGGQGTQVT VSS | |
| 48 | caggtgcagctgcaagaaagcggagggggcctggtccag ccaggaggcagcctccggctaagttgcgaggccagcggc ttcacattcagtagatttggcatgacatgggtcaggcagg ccccaggtaaaggcgtggaatgggtaagcggcataagca gtctgggagacagcacactctacgccgattccgtgaaag gccgcttcaccatctctagagacaacgcaaaaaataccc tgtaccttcagatgaattcactcaagcccgaagacacgg ccgtttattactgcacaatcggcgggtcactaaacccagg cggacaggggacacaagtaacggttagtagc | Nucleic acid sequence of the nanobody against Albumine (Alb8) |
| 49 | gaagtgcagcttcaggctagcgggggaggcttggtgca ggctggggggtctctgagactctcctgtagcgcctctgtg cggacctttagcatctacgcaatgggctggttccgccagg ctccagggaaggaacgcgagttcgtcgcgggcatcaaca gaagtggtgacgtaacaaaatacgccgatttcgtaaagg ggcgattcagcatctccagagacaacgccaagaacatgg tgtatctgcagatgaacagcttgaaacctgaggacacag ccctgtattactgcgccgcgacctgggcgtacgacaccg tgggcgcgctgaccagcggctataatttttggggccaag gcacccaggtcaccgtgagctca | Nucleic acid sequence of the nanobody against Transferin receptor (Tfr) |
| 50 | caggtgcagcttgtacagtctgggggaggcttggtgcag gctggggggtctctgagactctcctgtgcagcctctgtaa ggttcttcagtattaataccatgggctggtatcgccagg ctccagggaagcagcgcgagttggtcgcagatattacta gtagtggtagcacaaactatgcagactccgggaagggc cgattcaccatctccagagacaacgccaagaacacggt gtatctgcagatgaacaggttgaaacctgaggacacag ccgtctattactgccatgcggattacaaatacacaaccc ataacacggcctggggccagggtacccaggtcaccgtc tcctcaggtgggggtggcagcggtggcggcggtagcggg | Nucleic acid sequence of the biparatopic nanobody IGF-1267 |
| | ggcggtggcagccaggtgcaattggtgcagtctggggg aggattggtgcaggctgggggctctctgagactctcctg tgcagcctctggacgcacctttagtagcttcgccatggc ctggttccgccaggctccagggaaggagcgtgagtttgt agcagctattagctggagtggtagtacaggccatgcaga ctctgtgaagggccgattcaccatcaccagagacaacgc caagaacacggtgtttctgcaaatgaacagcctgaaacc tgaggacacggccgtttattactgtgcactaggaaagggg agtagttactactacagtcctagtgggtatgactactggg gccaggggacccaggtcaccgtctcctcaggtgggggtg gcagcggtggcggcggtagcgaagtgcagcttcaggct agcgggggaggcttggtgcaggctggggggtctctgaga ctctcctgtagcgcctctgtgcggacctttagcatctacgc aatgggctggttccgccaggctccagggaaggaacgcga gttcgtcgcgggcatcaacagaagtggtgacgtaacaaa atacgccgatttcgtaaaggggcgattcagcatctccaga gacaacgccaagaacatggtgtatctgcagatgaacagc ttgaaacctgaggacacagccctgtattactgcgccgcga cctgggcgtacgacaccgtgggcgcgctgaccagcggcta taatttttggggccaaggcacccaggtcaccgtgagctca | |
| 51 | caggtgcagcttgtacagtctgggggaggcttggtgcagg ctggggggtctctgagactctcctgtgcagcctctgtaagg ttcttcagtattaataccatgggctggtatcgccaggctcc agggaagcagcgcgagttggtcgcagatattactagtagt ggtagcacaaactatgcagactccgggaagggccgattc accatctccagagacaacgccaagaacacggtgtatctg cagatgaacaggttgaaacctgaggacacagccgtctat tactgccatgcggattacaaatacacaacccataacacg gcctggggccagggtacccaggtcaccgtctcctcagaa ccgaagatcccgcagccgcagccaaaacctcagccgcag ccacagccgcaacctcaaccgaaaccgcaaccaaaaccg gaacctcaggtgcaattggtgcagtctgggggaggattgg tgcaggctgggggctctctgagactctcctgtgcagcctct ggacgcacctttagtagcttcgccatggcctggttccgcca ggctccagggaaggagcgtgagtttgtagcagctattagct | Nucleic acid sequence of the biparatopic nanobody IGF-1268 |
| | ggagtggtagtacaggccatgcagactctgtgaagggccg attcaccatcaccagagacaacgccaagaacacggtgttt ctgcaaatgaacagcctgaaacctgaggacacggccgttt attactgtgcactaggaaaggggagtagttactactacagt cctagtgggtatgactactggggccaggggacccaggtca ccgtctcctcaggtgggggtggcagcggtggcggcggtag cgaagtgcagcttcaggctagcgggggaggcttggtgca ggctggggggtctctgagactctcctgtagcgcctctgtgc ggacctttagcatctacgcaatgggctggttccgccaggct ccagggaaggaacgcgagttcgtcgcgggcatcaacagaa gtggtgacgtaacaaaatacgccgatttcgtaaaggggcga ttcagcatctccagagacaacgccaagaacatggtgtatct gcagatgaacagcttgaaacctgaggacacagccctgtatt actgcgccgcgacctgggcgtacgacaccgtgggcgcgctg accagcggctataatttttggggccaaggcacccaggtcac cgtgagctca | |
| 52 | caggtgcagcttgtacagtctgggggaggcttggtgcaggc tggggggtctctgagactctcctgtgcagcctctgtaaggtt cttcagtattaataccatgggctggtatcgccaggctccag ggaagcagcgcgagttggtcgcagatattactagtagtggt agcacaaactatgcagactccgggaagggccgattcacca tctccagagacaacgccaagaacacggtgtatctgcagat gaacaggttgaaacctgaggacacagccgtctattactgc catgcggattacaaatacacaacccataacacggcctggg gccagggtacccaggtcaccgtctcctcaggtgggggtgg cagcggtggcggcggtagcgggggcggtggcagccaggtg caattggtgcagtctgggggaggattggtgcaggctgggg gctctctgagactctcctgtgcagcctctggacgcaccttta gtagcttcgccatggcctggttccgccaggctccagggaag gagcgtgagtttgtagcagctattagctggagtggtagtac aggccatgcagactctgtgaagggccgattcaccatcacca gagacaacgccaagaacacggtgtttctgcaaatgaacagc ctgaaacctgaggacacggccgtttattactgtgcactagga aaggggagtagttactactacagtcctagtgggtatgactac tggggccaggggacccaggtcaccgtctcctcaggtggggg | Nucleic acid sequence of the biparatopic nanobody IGF-1269 |
| | tggcagcggtggcggcggtagccaggtgcagctgcaagaaa gcggagggggcctggtccagccaggaggcagcctccggcta agttgcgaggccagcggcttcacattcagtagatttggcatg acatgggtcaggcaggccccaggtaaaggcgtggaatgggt aagcggcataagcagtctgggagacagcacactctacgccg attccgtgaaaggccgcttcaccatctctagagacaacgcaa aaaataccctgtaccttcagatgaattcactcaagcccgaag acacggccgtttattactgcacaatcggcgggtcactaaacc caggcggacaggggacacaagtaacggttagtagc | |
| 53 | caggtgcagcttgtacagtctgggggaggcttggtgcaggct ggggggtctctgagactctcctgtgcagcctctgtaaggttct tcagtattaataccatgggctggtatcgccaggctccaggga agcagcgcgagttggtcgcagatattactagtagtggtagca caaactatgcagactccgggaagggccgattcaccatctcc agagacaacgccaagaacacggtgtatctgcagatgaaca ggttgaaacctgaggacacagccgtctattactgccatgcg gattacaaatacacaacccataacacggcctggggccaggg tacccaggtcaccgtctcctcagaaccgaagatcccgcagc cgcagccaaaacctcagccgcagccacagccgcaacctcaa ccgaaaccgcaaccaaaaccggaacctcaggtgcaattggt gcagtctgggggaggattggtgcaggctgggggctctctgag actctcctgtgcagcctctggacgcacctttagtagcttcgcc atggcctggttccgccaggctccagggaaggagcgtgagttt gtagcagctattagctggagtggtagtacaggccatgcagac tctgtgaagggccgattcaccatcaccagagacaacgccaa gaacacggtgtttctgcaaatgaacagcctgaaacctgagga cacggccgtttattactgtgcactaggaaaggggagtagttac tactacagtcctagtgggtatgactactggggccaggggacc caggtcaccgtctcctcaggtgggggtggcagcggtggcggc ggtagccaggtgcagctgcaagaaagcggagggggcctggt ccagccaggaggcagcctccggctaagttgcgaggccagcg gcttcacattcagtagatttggcatgacatgggtcaggcaggc cccaggtaaaggcgtggaatgggtaagcggcataagcagtct gggagacagcacactctacgccgattccgtgaaaggccgctt caccatctctagagacaacgcaaaaaataccctgtaccttca | Nucleic acid sequence of the biparatopic nanobody IGF-1270 |
| | gatgaattcactcaagcccgaagacacggccgtttattactg cacaatcggcgggtcactaaacccaggcggacaggggacac aagtaacggttagtagc | |

### Monovalent nanobodies

As used herein the term "single domain antibody" has its general meaning in the art and refers to the single heavy chain variable domain of antibodies of the type that can be found in Camelid mammals which are naturally devoid of light chains. Such single domain antibody are also called VHH or "nanobody^{®}". For a general description of single domain antibodies, reference is made to EP 0 368 684, Ward et al. (Nature 1989 Oct 12; 341 (6242): 544-6), Holt et al., Trends Biotechnol., 2003, 21 (11):484-490; and WO 06/030220, WO 06/003388. The amino acid sequence and structure of a single domain antibody can be considered to be comprised of four framework regions or "FRs" which are referred to in the art and herein as "Framework region 1" or "FRl "; as "Framework region 2" or "FR2"; as "Framework region 3 " or "FR3"; and as "Framework region 4" or "FR4" respectively; which framework regions are interrupted by three complementary determining regions or "CDRs", which are referred to in the art as "Complementarity Determining Region 1 or "CDR1"; as "Complementarity Determining Region 2" or "CDR2" and as "Complementarity Determining Region 3" or "CDR3", respectively. Accordingly, the single domain antibody can be defined as an amino acid sequence with the general structure : FR1 - CDR1 - FR2 - CDR2 - FR3 - CDR3 - FR4 in which FR1 to FR4 refer to framework regions 1 to 4 respectively, and in which CDR1 to CDR3 refer to the complementarity determining regions 1 to 3. In the context of the invention, the amino acid residues of the single domain antibody are numbered according to the general numbering for VH domains given by the International ImMunoGeneTics information system aminoacid numbering (http://imgt.cines.fr/).

In particular, an isolated single domain antibody ("IGF-1159 derivative") used in the present invention comprises a CDR1 having the sequence set forth as SEQ ID NO:1, a CDR2 having the sequence set forth as SEQ ID NO:2 and a CDR3 having the sequence set forth as SEQ ID NO:3.

In particular, an isolated single domain antibody ("IGF-1160 derivative") used in the present invention comprises a CDR1 having the sequence set forth as SEQ ID NO:4, a CDR2 having the sequence set forth as SEQ ID NO:5 and a CDR3 having the sequence set forth as SEQ ID NO:6.

In particular, an isolated single domain antibody (" IGF-1164 derivative") used in the present invention comprises a CDR1 having the sequence set forth as SEQ ID NO:7, a CDR2 having the sequence set forth as SEQ ID NO:8 and a CDR3 having the sequence set forth as SEQ ID NO:9.

In particular, an isolated single domain antibody ("IGF-1163 derivative") used in the present invention comprises a CDR1 having the sequence set forth as SEQ ID NO:10, a CDR2 having the sequence set forth as SEQ ID NO:11 and a CDR3 having the sequence set forth as SEQ ID NO:12.

In particular an isolated single domain antibody ("IGF-1166 derivative") used in the present invention comprises a CDR1 having the sequence set forth as SEQ ID NO:13, a CDR2 having the sequence set forth as SEQ ID NO:14 and a CDR3 having the sequence set forth as SEQ ID NO:15.

In particular, an isolated single domain antibody ("IGF-1168 derivative") used in the present invention comprises a CDR1 having the sequence set forth as SEQ ID NO:16, a CDR2 having the sequence set forth as SEQ ID NO:17 and a CDR3 having the sequence set forth as SEQ ID NO:18.

In a particular embodiment, the isolated single domain antibody directed against mGluR2 used as a biomolecule transporter according to the invention comprises :
a CDR1 having a sequence set forth as SEQ ID NO:1, a CDR2 having a sequence set forth as SEQ ID NO:2 and a CDR3 having a sequence set forth as SEQ ID NO:3; or
a CDR1 having a sequence set forth as SEQ ID NO 4, a CDR2 having a sequence set forth as SEQ ID NO:5 and a CDR3 having a sequence set forth as SEQ ID NO:6 ; or
a CDR1 having a sequence set forth as SEQ ID NO:7 , a CDR2 having a sequence set forth as SEQ ID NO:8 and a CDR3 having a sequence set forth as SEQ ID NO:9; or
a CDR1 having a sequence set forth as SEQ ID NO:10 a CDR2 having a sequence set forth as SEQ ID NO:11 and a CDR3 having a sequence set forth as SEQ ID NO:12 or
a CDR1 having a sequence set forth as SEQ ID NO:13 a CDR2 having a sequence set forth as SEQ ID NO:14 and a CDR3 having a sequence set forth as SEQ ID NO:15; or
a CDR1 having a sequence set forth as SEQ ID NO:16, a CDR2 having a sequence set forth as SEQ ID NO:17 and a CDR3 having a sequence set forth as SEQ ID NO:18.

In some embodiments, the isolated single domain antibody used according to the invention has at least 85%, preferably 90%, more preferably 95% and even preferably 100% identity with the sequence set forth as SEQ ID NO:19 ("IGF-1159 nanobody").

In some embodiments, the isolated single domain antibody used according to the invention has at least 85%, preferably 90%, more preferably 95% and even preferably 100% identity with the sequence set forth as SEQ ID NO:20 ("IGF-1160 nanobody").

In some embodiments, the isolated single domain antibody used according to the invention has at least 85%, preferably 90%, more preferably 95% and even preferably 100% identity with the sequence set forth as SEQ ID NO:21 ("IGF-1164 nanobody").

In some embodiments, the isolated single domain antibody used according to the invention has at least 85%, preferably 90%, more preferably 95% and even preferably 100% identity with the sequence set forth as SEQ ID NO:22 ("IGF-1163 nanobody").

In some embodiments, the isolated single domain antibody used according to the invention has at least 85%, preferably 90%, more preferably 95% and even preferably 100% identity with the sequence set forth as SEQ ID NO:23 ("IGF-1166 nanobody").

In some embodiments, the isolated single domain antibody used according to the invention has at least 85%, preferably 90%, more preferably 95% and even preferably 100% identity with the sequence set forth as SEQ ID NO:24 ("IGF-1168 nanobody").

In a particular embodiment, the single domain antibody directed against mGluR2 used as a biomolecule transporter according to the invention has at least 85% identity with anyone of the sequences set forth as SEQ ID NO:19 to SEQ ID NO: 21, preferably at least 90% identity, more preferably at least 95% identity, and even 100% identity with anyone of the sequences set forth as SEQ ID NO:19 to SEQ ID NO: 21.

In a particular and preferred embodiment, the single domain antibody directed against mGluR2 used as a biomolecule transporter according to the invention has the sequence set forth as SEQ ID NO:19.

In another particular and preferred embodiment, the single domain antibody directed against mGluR2 used as a biomolecule transporter according to the invention has the sequence set forth as SEQ ID NO:21.

According to the invention a first amino acid sequence having at least 85% of identity with a second amino acid sequence means that the first sequence has 85; 86; 87; 88; 89; 90; 91; 92; 93; 94; 95; 96; 97; 98; or 99% of identity with the second amino acid sequence. Amino acid sequence identity is typically determined using a suitable sequence alignment algorithm and default parameters, such as BLAST P (Karlin and Altschul, 1990).

The sequence identity is calculated by sequence alignment according to known methods in the art.

To determine the percent identity of two amino acids sequences, the sequences are aligned for optimal comparison. For example, gaps can be introduced in the sequence of a first amino acid sequence for optimal alignment with the second amino acid sequence. The amino acid residues at corresponding amino acid positions are then compared. When a position in the first sequence is occupied by the same amino acid residue as the corresponding position in the second sequence, the molecules are identical at that position. The percent identity between the two sequences is a function of the number of identical positions shared by the sequences. Hence % identity = number of identical positions/total number of overlapping positions X 100.

In this comparison the sequences can be the same length or can be different in length. Optimal alignment of sequences for determining a comparison window may be conducted by the local homology algorithm of Smith and Waterman (J. Theor. Biol., 1981), by the homology alignment algorithm of Needleman and Wunsch (J. Mol. Biol, 1972), by the search for similarity via the method of Pearson and Lipman (Proc. Natl. Acad. Sci. U.S.A., 1988), by computerized implementations of these algorithms (GAP, BESTFIT, FASTA and TFASTA in the Wisconsin Genetics Software Package Release 7.0, Genetic Computer Group, 575, Science Drive, Madison, Wisconsin) or for instance using publicly available computer software such as BLAST[2]. When using such software, the default parameters, e.g for gap penalty or extension penalty, are preferably used. The best alignment (i.e. resulting in the highest percentage of identity over the comparison window) generated by the various methods is selected.

In some embodiments, the single domain antibody is a "humanized" single domain antibody. As used herein the term "humanized" refers to a single domain antibody of the invention wherein an amino acid sequence that corresponds to the amino acid sequence of a naturally occurring VHH domain has been "humanized", i.e. by replacing one or more amino acid residues in the amino acid sequence of said naturally occurring VHH sequence (and in particular in the framework sequences) by one or more of the amino acid residues that occur at the corresponding position(s) in a VH domain from a conventional chain antibody from a human being. Methods for humanizing single domain antibodies are well known in the art. Typically, the humanizing substitutions should be chosen such that the resulting humanized single domain antibodies still retain the favourable properties of single domain antibodies of the invention. The one skilled in the art is able to determine and select suitable humanizing substitutions or suitable combinations of humanizing substitutions. For example, the single domain antibodies of the invention may be suitably humanized at any framework residue provided that the single domain antibodies remain soluble and do not significantly loss their affinity for mGluR2.

A further aspect of the invention refers to a polypeptide comprising at least one single domain antibody of the invention for use as a biomolecule transporter crossing the BBB.

Typically, the polypeptide of the invention comprises a single domain antibody of the invention, which is fused at its N terminal end, at its C terminal end, or both at its N terminal end and at its C terminal end to at least one further amino acid sequence, i.e. so as to provide a fusion protein. According to the invention the polypeptides that comprise a sole single domain antibody are referred to herein as "monovalent" polypeptides. Polypeptides that comprise or essentially consist of two or more single domain antibodies according to the invention are referred to herein as "multivalent" polypeptides.

In a particular embodiment, the polypeptide comprises two single domain antibodies and is referred herein as 'biparatopic polypeptide' or 'biparatopic nanobody'.

In another particular embodiment, the polypeptide comprises three single domain antibodies and is referred herein as 'triparatopic polypeptide' or 'triparatopic nanobody'.

Thus, in some embodiments, the polypeptide of the invention may also provide at least one further binding domain directed against any desired protein, polypeptide, antigen, antigenic determinant or epitope. Said binding domain is directed against the same protein, polypeptide, antigen, antigenic determinant or epitope for which the single domain antibody of the invention is directed against, or may be directed against a different protein, polypeptide, antigen, antigenic determinant or epitope from the single domain antibody of the invention.

In some embodiments, the further binding domain is directed against a serum protein so that the half-life of the single domain antibody is increased. Typically, said serum protein is albumin.

In a particular embodiment, the polypeptide is a bivalent or biparatopic polypeptide.

### Bivalent (biparatopic) nanobodies

The term "bivalent" or "biparatopic" polypeptide or nanobody according to the invention means a nanobody comprising a single domain antibody and a second single domain antibody as herein defined, wherein these two single domain antibodies are capable of binding to two different epitopes of one antigen (e.g. mGluR2), which epitopes are not normally bound at the same time by one monospecific immunoglobulin, such as e.g. a conventional antibody or one single domain antibody.

In a particular embodiment, the biparatopic polypeptide used as a biomolecule transporter according to the invention comprises :
(i) one single domain antibody having
   a CDR1 having a sequence set forth as SEQ ID NO:1, a CDR2 having a sequence set forth as SEQ ID NO:2 and a CDR3 having a sequence set forth as SEQ ID NO:3; or
   a CDR1 having a sequence set forth as SEQ ID NO:4, a CDR2 having a sequence set forth as SEQ ID NO:5 and a CDR3 having a sequence set forth as SEQ ID NO: 6; or
   a CDR1 having a sequence set forth as SEQ ID NO:7, a CDR2 having a sequence set forth as SEQ ID NO:8 and a CDR3 having a sequence set forth as SEQ ID NO:9;
   preferably
      a CDR1 having a sequence set forth as SEQ ID NO:1, a CDR2 having a sequence set forth as SEQ ID NO:2 and a CDR3 having a sequence set forth as SEQ ID NO:3;
      and
(ii) another single domain antibody having
   a CDR1 having a sequence set forth as SEQ ID NO:10, a CDR2 having a sequence set forth as SEQ ID NO:11 and a CDR3 having a sequence set forth as SEQ ID NO:12; or
   a CDR1 having a sequence set forth as SEQ ID NO:13, a CDR2 having a sequence set forth as SEQ ID NO:14 and a CDR3 having a sequence set forth as SEQ ID NO:15; or
   a CDR1 having a sequence set forth as SEQ ID NO:16, a CDR2 having a sequence set forth as SEQ ID NO:17 and a CDR3 having a sequence set forth as SEQ ID NO:18.

In a particular embodiment, the biparatopic polypeptide used as a biomolecule transporter in the present invention comprises (i) one single domain antibody having a CDR1 having a sequence set forth as SEQ ID NO:1, a CDR2 having a sequence set forth as SEQ ID NO:2 and a CDR3 having a sequence set forth as SEQ ID NO:3; and (ii) another single domain antibody having a CDR1 having a sequence set forth as SEQ ID NO:16, a CDR2 having a sequence set forth as SEQ ID NO:17 and a CDR3 having a sequence set forth as SEQ ID NO:18.

In another particular embodiment, the biparatopic polypeptide used in the present invention comprises (i) one single domain antibody having a CDR1 having a sequence set forth as SEQ ID NO:1, a CDR2 having a sequence set foth as SEQ ID NO:2 and a CDR3 having a sequence set forth as SEQ ID NO:3; and (ii) another single domain antibody having a CDR1 having a sequence set forth as SEQ ID NO:10, a CDR2 having a sequence set forth as SEQ ID NO:11 and a CDR3 having a sequence set forth as SEQ ID NO:12.

In another particular embodiment, the biparatopic polypeptide used as a biomolecule transporter according to the present invention comprises (i) one single domain antibody having a CDR1 having a sequence set forth as SEQ ID NO:1 a CDR2 having a sequence set foth as SEQ ID NO:2 and a CDR3 having a sequence set forth as SEQ ID NO:3; and (ii) another single domain antibody having a CDR1 having a sequence set forth as SEQ ID NO:13, a CDR2 having a sequence set foth as SEQ ID NO:14 and a CDR3 having a sequence set forth as SEQ ID NO:15.

In a particular embodiment, the biparatopic polypeptide used as a biomolecule transporter according the present invention comprises one single domain antibody having at least 85% identity, preferably at least 90% identity, more preferably at least 95% identity, and even 100% identity with the sequence with the sequence set forth as SEQ ID NO:19, and another single domain antibody having at least 85% identity, preferably at least 90% identity, more preferably at least 95% identity, and even 100% identity with the sequence with the sequence set forth as SEQ ID NO:24.

In a particular embodiment, the biparatopic polypeptide used as a biomolecule transporter according the present invention comprises one single domain antibody having at least 85% identity, preferably at least 90% identity, more preferably at least 95% identity, and even 100% identity with the sequence with the sequence set forth as SEQ ID NO:19, and another single domain antibody having at least 85% identity, preferably at least 90% identity, more preferably at least 95% identity, and even 100% identity with the sequence with the sequence set forth as SEQ ID NO:22.

In a particular embodiment, the biparatopic polypeptide used as a biomolecule transporter according the present invention comprises one single domain antibody having at least 85% identity, preferably at least 90% identity, more preferably at least 95% identity, and even 100% identity with the sequence with the sequence set forth as SEQ ID NO:19, and another single domain antibody having at least 85% identity, preferably at least 90% identity, more preferably at least 95% identity, and even 100% identity with the sequence with the sequence set forth as SEQ ID NO:23.

### Linker

In some embodiments, the two single domain antibodies of the biparatopic nanobody used in the present invention can be linked to each other directly (i.e. without use of a linker) or via a linker. The linker is typically a linker peptide and will, according to the invention, be selected so as to allow binding of the two single domain antibodies to each of their at least two different epitopes of mGluR2. Suitable linkers inter alia depend on the epitopes and, specifically, the distance between the epitopes on mGluR2 to which the single domain antibodies bind, and will be clear to the skilled person based on the disclosure herein, optionally after some limited degree of routine experimentation.

Suitable linkers are described herein in connection with specific nanobodies of the invention and may - for example and without limitation - comprise an amino acid sequence, which amino acid sequence preferably has a length of 9 or more amino acids, more preferably at least 17 amino acids, such as about 20 to 40 amino acids. However, the upper limit is not critical but is chosen for reasons of convenience regarding e.g. biopharmaceutical production of such nanobody. The linker sequence may be a naturally occurring sequence or a non-naturally occurring sequence. If used for therapeutical purposes, the linker is preferably non-immunogenic in the subject to which the anti-mGluR2 polypeptide of the invention is administered. One useful group of linker sequences are linkers derived from the hinge region of heavy chain antibodies as described in WO 96/34103 and WO 94/04678. Other examples are poly-alanine linker sequences such as Ala-Ala-Ala. Another preferred example of linker sequence (L1) is EPKIPQPQPKPQPQPQPQPQPKPQPKPEP (SEQ ID NO:25, also named Linker L1 or 'HcAb').

Further preferred examples of linker sequences are Gly/Ser linkers of different length including (gly4ser)3 also named (GGGGS)₃, (gly4ser)4, (gly4ser), (gly3ser), gly3, and (gly3ser2)3.
SEQ ID NO:26 (GGGGS GGGGS GGGGS) also named Linker L2 or 'GS'
Linker L3: SEQ ID NO:27 (GGGGS GGGGS GGGGS GGGGS)
Linker L4: SEQ ID NO: 28 (GGGGS)
Linker L5: SEQ ID NO:29 (GGS)
Linker L6: SEQ ID NO:30 (GGG)
Linker L7: SEQ ID NO:31 (GGGSS GGGSS GGGSS)

In a particular embodiment, the linker sequence has sequence SEQ ID NO:26 (GGGGS GGGGS GGGGS).

In another a preferred embodiment, the first and second single domains are linked with a linker sequence having at least 80% of identity with the sequence set forth as SEQ ID NO:25, preferably 100% identity with SEQ ID NO: 25 (EPKIPQPQPKPQPQPQPQPQPKPQPKPEP).

The construction of the biparatopic nanobody may be either in a sense or a reverse sense, meaning that for each combination two single domain antibodies, we have 2 possible constructs.

The following table 2 lists the different contructs prepare in the context of the invention, wherein :
- the amino acid sequences of IGF-11xx nanobodies are defined as above ;
- the linkers are respectively 'HcAb' (EPKIPQPQPKPQPQPQPQPQPKPQPKPEP = SEQ ID NO:25) or 'GS' (GGGGS GGGGS GGGGS = SEQ ID NO: 26), and
- IGF-1159, IGF-1160 and IGF-1164 respectively are neutral nanobodies, mGlu2 specific; and IGF-1163, IGF-1166 and IGF-1168 respectively are PAM nanobodies, mGlu2 specific.

Advantageously, the biparatopic nanobody used in the invention combines a neutral nanobody and a PAM nanobody.

In a particular and preferred embodiment, the neutral nanobody used in the present invention is IGF-1159.

In a particular and preferred embodiment, the PAM antibody used in the present invention is IGF-1168.

So in a particular and preferred embodiment, the biparatopic nanobody used in the present invention comprises IGF-1159 nanobody and IGF-1168, linked by a linker as defined above, and in particular the linker of SEQ ID NO:25, forming the biparatopic nanobody IGF-1202.

**Table 2 illustrates a library of mGlu2 nanobodies generated with the non-limitative nanobodies and linkers illustrated in the invention**

| | |
|---|---|
| IGF-1159_{GS}**IGF-1159** | IGF-1191 |
| IGf-1159_{HcAb}**IGF-1159** | IGF-1192 |
| IGF-1160_{GS}**IGF-1159** | IGF-1193 |
| IGF-1160_{HcAb}**IGF-1159** | IGF-1194 |
| IGF-1163_{GS}**IGF-1159** | IGF-1195 |
| IGF-1163_{HcAb}**IGF-1159** | IGF-1196 |
| IGF-1164_{GS}**IGF-1159** | IGF-1197 |
| IGF-1164_{HcAb}**IGF-1159** | IGF-1198 |
| IGF-1166_{GS}**IGF-1159** | IGF-1199 |
| IGF-1166 _{HcAb}**IGF-1159** | IGF-1200 |
| IGF-1168 _{GS}**IGF-1159** | IGF-1201 |
| IGF-1168 _{HcAb} **IGF-1159** | **IGF-1202** |
| | |
| IGF-1159_{GS}**IGF-1160** | IGF-1203 |
| IGF-1159_{HcAb}**IGF-1159** | IGF-1204 |
| IGF-1160_{GS}**IGF-1160** | IGF-1205 |
| IGF-1160_{HcAb}**IGF-1160** | IGF-1206 |
| IGF-1163_{GS}**IGF-1160** | IGF-1207 |
| IGF-1163_{HcAb}**IGF-1160** | IGF-1208 |
| IGF-1164_{GS}**IGF-1160** | IGF-1209 |
| IGF-1164_{HcAb}**IGF-1160** | IGF-1210 |
| IGF-1166_{GS}**IGF-1160** | IGF-1211 |
| IGF-1166_{HcAb}**IGF-1160** | IGF-1212 |
| IGF-1168_{GS}**IGF-1160** | IGF-1213 |
| IGF-1168_{HcAb}**IGF-1160** | IGF-1214 |
| | |
| IGF-1159_{GS}**IGF-1163** | IGF-1215 |
| IGF-1159_{HcAb}**IGF-1163** | IGF-1216 |
| IGF-1160_{GS}**IGF-1163** | IGF-1217 |
| IGF-1160_{HcAb}**IGF-1163** | IGF-1218 |
| IGF-1163_{GS}**IGF-1163** | IGF-1219 |
| IGF-1163_{HcAb}**IGF-1163** | IGF-1220 |
| IGF-1164_{GS}**IGF-1163** | IGF-1221 |
| IGF-1164_{HcAb}**IGF-1163** | IGF-1222 |
| IGF-1166_{GS}**IGF-1163** | IGF-1223 |
| IGF-1166_{HcAb}**IGF-1163** | IGF-1224 |
| IGF-1168_{GS}**IGF-1163** | IGF-1225 |
| IGF-1168_{HcAb}**IGF-1163** | IGF-1226 |
| | |
| IGF-1159_{GS}**IGF-1164** | IGF-1227 |
| IGF-1159_{HcAb}**IGF-1164** | IGF-1228 |
| IGF-1160_{GS}**IGF-1164** | IGF-1229 |
| IGF-1160_{HcAb}**IGF-1164** | IGF-1230 |
| IGF-1163_{GS}**IGF-1164** | IGF-1231 |
| IGF-1163_{HcAb}**IGF-1164** | IGF-1232 |
| IGF-1164_{GS}**IGF-1164** | IGF-1233 |
| IGF-1164_{HcAb}**IGF-1164** | IGF-1234 |
| IGF-1166_{GS}**IGF-1164** | IGF-1235 |
| IGF-1166_{HcAbI}**GF-1164** | IGF-1236 |
| IGF-1168_{GS}**IGF-1164** | IGF-1237 |
| IGF-1168_{HcAbI}**GF-1164** | IGF-1238 |
| | |
| IGF-1159_{GS}**IGF-1166** | IGF-1239 |
| IGF-1159_{HcAb}**IGF-1166** | IGF-1240 |
| IGF-1160_{GS}**IGF-1166** | IGF-1241 |
| IGF-1160_{HcAb}**IGF-1166** | IGF-1242 |
| IGF-1163_{GS}**IGF-1166** | IGF-1243 |
| IGF-1163_{HcAb}**IGF-1166** | IGF-1244 |
| IGF-1164_{GS}**IGF-1166** | IGF-1245 |
| IGF-1164_{HcAb}**IGF-1166** | IGF-1246 |
| IGF-1166_{GS}**IGF-1166** | IGF-1247 |
| IGF-1166_{HcAb}**IGF-1166** | IGF-1248 |
| IGF-1168_{GS}**IGF-1166** | IGF-1249 |
| IGF-1168_{HcAb}**IGF-1166** | IGF-1250 |
| | |
| IGF-1159_{GS}**IGF-1168** | IGF-1251 |
| IGF-1159_{HcAb}**IGF-1168** | IGF-1252 |
| IGF-1160_{GS}**IGF-1168** | IGF-1253 |
| IGF-1160_{HcAb}**IGF-1168** | IGF-1254 |
| IGF-1163_{GS}**IGF-1168** | IGF-1255 |
| IGF-1163_{HcAb}**IGF-1168** | IGF-1256 |
| IGF-1164_{GS}**IGF-1168** | IGF-1257 |
| IGF-1164_{HcAb}**IGF-1168** | IGF-1258 |
| IGF-1166_{GS}**IGF-1168** | IGF-1259 |
| IGF-1166_{HcAb}**IGF-1168** | IGF-1260 |
| IGF-1168_{GS}**IGF-1168** | IGF-1261 |
| IGF-1168_{HcAb}**IGF-1168** | IGF-1262 |
| **IGF-1168-Tfr** | IGF-1263 and IGF-1264 |
| **IGF-1168-Alb8** | IGF-1265 and IGF-1266 |
| IGF-1168_{GS}**IGF-1159 -Tfr** | IGF-1267 |
| IGF-1168_{HcAb}**IGF-1159-Tfr** | IGF-1268 |
| IGF-1168_{GS}**IGF-1159-Alb8** | IGF-1269 |
| IGF-1168_{HcAb}**IGF-1159-Alb8** | IGF-1270 |

In a particular embodiment, the following constructs are of particular interest:

| | |
|---|---|
| IGF-1168 _{GS}**IGF-1159** | IGF-1201 |
| IGF-1168 _{HcAb} **IGF-1159** | **IGF-1202** |
| **IGF-1168-Tfr** | IGF-1263 and IGF-1264 |
| **IGF-1168-Alb8** | IGF-1265 and IGF-1266 |
| IGF-1168GSIGF-1159-Alb8 | **IGF-1269** |
| IGF-1168HcAblGF-1159-Alb8 | **IGF-1270** |

So in a particular embodiment, the biparatopic nanobody used in the present invention is selected in the group consisting of:
SEQ ID NO:19 (IGF-1159)- linker- SEQ ID NO: 24 (IGF-1168)
SEQ ID NO:24 (IGF1168)- linker- SEQ ID NO: 19 (IGF-1159)
SEQ ID NO:19 (IGF-1159)- linker - SEQ ID NO:22 (IGF-1163)
SEQ ID NO:22 (IGF-1163)- linker - SEQ ID NO:19 (IGF-1159)
SEQ ID NO:19 (IGF-1159)- linker - SEQ ID NO:23 (IGF-1166)
SEQ ID NO:23 (IGF-1166)- linker - SEQ ID NO:19 (IGF-1159)

In a particular and preferred embodiment, the biparatopic nanobody used in the invention comprises the construct SEQ ID NO:24 (IGF-1168) - linker- SEQ ID NO: 19 (IGF-1159. The linker is preferably EPKIPQPQPKPQPQPQPQPQPKPQPKPEP (SEQ ID NO: 25).

In particular, the biparatopic nanobody used in the invention comprises the amino acid sequence set forth as SEQ ID NO:32 also named IGF1202 corresponding to the construct IGF-1168 HcAb IGF-1159).

In another particular embodiment, the biparatopic nanobody used in the present invention comprises the amino acid sequence set forth as SEQ ID NO:33 ((also named IGF1202-tag6xHIS) .

In a particular and preferred embodiment, the biparatopic nanobody used in the invention comprises an amino-acid sequence having at least 80% of identity with the sequence set forth as SEQ ID NO:32 or SEQ ID NO:33.

By at least 80% of identity, it means 80, 81, 82, 83, 84, 85, 86, 87, 88, 89, 90, 91, 92, 93, 94, 95, 96, 97, 98, 99, or 100% identity with the said sequences.

In the case of substitution of one or more consecutive or non-consecutive amino acids, substitutions are preferred in which the substituted amino acids are replaced by "equivalent" amino acids. Here, the expression "equivalent amino acids" is meant to indicate any amino acids likely to be substituted for one of the structural amino acids without however modifying the biological activities of the corresponding nanobodies or biparatopic nanobodies and of those specific examples defined below. Equivalent amino acids can be determined either on their structural homology with the amino acids for which they are substituted or on the results of comparative tests of biological activity between the various nanobodies likely to be generated.

As a non-limiting example, **Table 3** below summarises the possible substitutions likely to be carried out without resulting in a significant modification of the biological activity of the corresponding modified antigen binding protein; inverse substitutions are naturally possible under the same conditions.

**Table 3**

| Original residue | Substitution(s) |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (E) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

### Other binding domains

Also, the two single domain antibodies that bind to mGluR2 may also be linked to each other via a third single domain antibody (in which the two single domain antibodies may be linked directly to the third domain antibody or via suitable linkers). Such a third single domain antibody may for example be a single domain antibody that provides an increased half-life. For example, the latter single domain antibody may be a single domain antibody that is capable of binding to a (human) serum protein such as (human) serum albumin, as further described herein. In some embodiments, two or more single domain antibodies that bind to mGluR2 are linked in series (either directly or via a suitable linker) and the third (single) single domain antibody (which may provide for increased half-life, as described above) is connected directly or via a linker to one of these two or more aforementioned single domain antibodies.

In a particular embodiment, the biparatopic nanobody comprises a further domain, such as a peptide, a nanobody or a fluorinated chain, and in particular a further domain directed against a serum protein, preferably serum albumin.

In a particular embodiment, the biparatopic nanobody of the invention also comprises at least one further binding domain directed against another antigen (i.e. different from mGluR2). In some embodiments, the further binding domain is directed against a serum protein so that the half-life of the biparatopic nanobody is increased. Typically, said serum protein is albumin. In a particular embodiment, the further binding domain is directed against albumin, and may be a peptide (Stork et al., 2009), a nanobody (Walker et al., 2010; Hutt et al., 2012) or a fluorinated chain (Gerbier et al., 2017).

In a particular and preferred embodiment, the biparatopic nanobody of the invention is IGF1201-Alb8 or IGF1202-Alb8 comprising an amino acid sequence having at least 80% of identity with the sequence set forth SEQ ID NO:46 or SEQ ID NO:47, preferably IGF1202-Alb8 of SEQ ID NO:47.

As disclosed above, by at least 80% of identity, it means 80, 81, 82, 83, 84, 85, 86, 87, 88, 89, 90, 91, 92, 93, 94, 95, 96, 97, 98, 99, or 100% identity with the said sequence.

### Production of the nanobodies

According to the invention, the nanobodies uses in the present invention may be produced by conventional automated peptide synthesis methods or by recombinant expression. General principles for designing and making proteins are well known to those of skill in the art. In particular, the nanobodies are prepared as disclosed in EP 3 164 415 A2

The nanobodies used in the present invention may be synthesized in solution or on a solid support in accordance with conventional techniques. Various automatic synthesizers are commercially available. The nanobodies of the invention may also be synthesized by solid-phase technology employing an exemplary peptide synthesizer such as a Model 433A from Applied Biosystems Inc. The purity of any given protein; generated through automated peptide synthesis or through recombinant methods may be determined using reverse phase HPLC analysis. Chemical authenticity of each peptide may be established by any method well known to those of skill in the art.

As an alternative to automated peptide synthesis, recombinant DNA technology may be employed wherein a nucleotide sequence which encodes a protein of choice is inserted into an expression vector, transformed or transfected into an appropriate host cell and cultivated under conditions suitable for expression as described herein below. Recombinant methods are especially preferred for producing longer polypeptides.

A variety of expression vector/host systems may be utilized to contain and express the peptide or protein coding sequence. These include but are not limited to microorganisms such as bacteria transformed with recombinant bacteriophage, plasmid or cosmid DNA expression vectors; yeast transformed with yeast expression vectors (Giga-Hama et al., 1999); insect cell systems infected with virus expression vectors (e.g., baculovirus, see Ghosh et al., 2002); plant cell systems transfected with virus expression vectors (e.g., cauliflower mosaic virus, CaMV; tobacco mosaic virus, TMV) or transformed with bacterial expression vectors (e.g., Ti or pBR322 plasmid; see e.g., Babe et al., 2000); or animal cell systems. Those of skill in the art are aware of various techniques for optimizing mammalian expression of proteins, see e.g., Kaufman, 2000; Colosimo et al., 2000. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells.

Exemplary protocols for the recombinant expression of the peptide substrates or fusion polypeptides in bacteria, yeast and other invertebrates are known to those of skill in the art and a briefly described herein below. Mammalian host systems for the expression of recombinant proteins also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing which cleaves a "prepro" form of the protein may also be important for correct insertion, folding and/or function. Different host cells such as CHO, HeLa, MDCK, 293, WI38, and the like have specific cellular machinery and characteristic mechanisms for such post-translational activities and may be chosen to ensure the correct modification and processing of the introduced, foreign protein.

In the recombinant production of the nanobodies of the invention, it would be necessary to employ vectors comprising polynucleotide molecules for encoding the first and second single domains antibodies of the invention. Methods of preparing such vectors as well as producing host cells transformed with such vectors are well known to those skilled in the art. The polynucleotide molecules used in such an endeavor may be joined to a vector, which generally includes a selectable marker and an origin of replication, for propagation in a host. These elements of the expression constructs are well known to those of skill in the art. Generally, the expression vectors include DNA encoding the given protein being operably linked to suitable transcriptional or translational regulatory sequences, such as those derived from a mammalian, microbial, viral, or insect genes. Examples of regulatory sequences include transcriptional promoters, operators, or enhancers, mRNA ribosomal binding domains, and appropriate sequences which control transcription and translation.

The terms "expression vector," "expression construct" or "expression cassette" are used interchangeably throughout this specification and are meant to include any type of genetic construct containing a nucleic acid coding for a gene product in which part or all of the nucleic acid encoding sequence is capable of being transcribed.

The choice of a suitable expression vector for expression of the nanobodies of the invention will of course depend upon the specific host cell to be used, and is well known by those of skill in the art.

In a particular embodiment, the nucleic acid sequence encodes for a biparatopic nanobody according to the invention, in particular a nucleic acid having at least 80% of identity with the sequence set forth as SEQ ID NO:40 (nucleic acid of biparatopic nanobody IGF1202) or SEQ ID NO:41 (nucleic acid of biparatopic nanobody IGF1202-tag6xHis).

By 'at least 80% of identity', it means at least 80; 81; 82; 83; 84; 85; 86; 87; 88; 89; 90; 91; 92; 93; 94; 95; 96; 97; 98; 99; or 100% identity with the sequence set forth as SEQ ID NO:40 or 41, according to the definition disclosed above.

Expression requires that appropriate signals be provided in the vectors, such as enhancers/promoters from both viral and mammalian sources that may be used to drive expression of the nucleic acids of interest in host cells. Usually, the nucleic acid being expressed is under transcriptional control of a promoter. A "promoter" refers to a DNA sequence recognized by the synthetic machinery of the cell, or introduced synthetic machinery, required to initiate the specific transcription of a gene. Nucleotide sequences are operably linked when the regulatory sequence functionally relates to the DNA encoding the proteins of interest (e.g., both single domains antibodies). Thus, a promoter nucleotide sequence is operably linked to a given DNA sequence if the promoter nucleotide sequence directs the transcription of the sequence.

In a particular embodiment, for large scale nanobody production, pHEN plasmid vector with the bivalent construct of the invention are transformed in E. coli BL21DE3 strain.

The fact that a biparatopic polypeptide comprising two single domain antibody targeting mGluR2 is able to cross the BBB is the demonstration that a single domain antibody targeting mGluR2 is able to cross the BBB with a small molecule such as another single domain antibody targeting mGluR2 or anyother biomolecule.

In particular, the biomolecule will have a molecular weight ranging from 15 to 60 kDa, in particular from 15 to 50 kDa, and particularly from 15 to 35 kDa.

In a particular embodiment, for use in biological and/or diagnostic analysis, the biomolecule linked to the single domain antibody directed against mGluR2 or a polypeptide comprising at least one single domain antibody directed against mGluR2 used in the present invention, is a detectable label.

The detectable label may be directly linked to the nanobody or polypeptide used in the present invention, or linked via a linker such as the ones disclosed above.

### Detectable labels

Suitable detectable labels include, for example, a radioisotope, a fluorescent label, a chemiluminescent label, an enzyme label, a bio luminescent label or colloidal gold. Methods of making and detecting such detectably-labeled immunoconjugates are well-known to those of ordinary skill in the art, and are described in more detail below. For instance, the detectable label can be a radioisotope that is detected by autoradiography. Isotopes that are particularly useful for the purpose of the present invention are ³H, ¹²⁵I, ¹³¹I, ³⁵S and ¹⁴C. Anti-mGluR2 immunoconjugates can also be labeled with a fluorescent compound. The presence of a fluorescently-labeled biparatopic nanobody of the present invention is determined by exposing the immuno conjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine and Alexa Fluor dyes. Alternatively, anti-mGluR2 immunoconjugates can be detectably labeled by coupling a biparatopic nanobody of the present invention to a chemiluminescent compound. The presence of the chemiluminescent-tagged immuno conjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester. Similarly, a bio luminescent compound can be used to label anti-mGluR2 immunoconjugates of the present invention. Bio luminescence is a type of chemiluminescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and aequorin. Alternatively, anti-mGluR2 immunoconjugates can be detectably labeled by linking an anti-mGluR2 biparatopic nanobody of the present invention to an enzyme. When the anti-mGluR2-enzyme conjugate is incubated in the presence of the appropriate substrate, the enzyme moiety reacts with the substrate to produce a chemical moiety which can be detected, for example, by spectrophotometric, fluorometric or visual means. Examples of enzymes that can be used to detectably label polyspecific immunoconjugates include β-galactosidase, glucose oxidase, peroxidase and alkaline phosphatase. Those of skill in the art will know of other suitable labels which can be employed in accordance with the present invention. The binding of marker moieties to anti-mGluR2 biparatopic nanobody of the present invention can be accomplished using standard techniques known to the art. Typical methodology in this regard is described by Kennedy et al., Clin. Chim. Acta 70: 1, 1976; Schurs et al., Clin. Chim. Acta 81 : 1, 1977; Shih et al., Int'U. Cancer 46: 1101, 1990; Stein et al, Cancer Res. 50: 1330, 1990; and Coligan, supra. Moreover, the convenience and versatility of immunochemical detection can be enhanced by using anti-mGluR2 biparatopic nanobody of the present invention that have been conjugated with avidin, streptavidin, and biotin. {See, e.g., Wilchek et al. (eds.), "Avidin-Biotin Technology," Methods In Enzymology (Vol. 184) (Academic Press 1990); Bayer et al., "Immunochemical Applications of Avidin-Biotin Technology," in Methods In Molecular Biology (Vol. 10) 149- 162 (Manson, ed., The Humana Press, Inc. 1992).

For example, said detection is performed by a TR-FRET method as described in WO2010125314. This method is based on the use of two antibodies labelled with a fluorophore donor or acceptor compatible with TR-FRET measurement. It can be either the same single domain antibody specific of the active conformation, or two different antibodies, such as two single domain antibodies specific of the active conformation, or one antibody specific of the active conformation and the other one not specific of the active conformation.

In a particular and preferred embodiment, the detectable label is selected from the group consisting of radioisotope labels, fluorescent labels, chemiluminescent labels, enzyme labels, and bio luminescent labels.

### Biological and diagnostic analysis

In a particular embodiment, the isolated single domain antibody directed against mGluR2 or the polypeptide comprising at least one single domain antibody directed against mGluR2 as defined above and linked to at least one detectable label, is used in biological analysis and/or diagnostic analysis.

By 'biological analysis' according to the invention, it means in particular the transfer into the brain of labelled molecules for PET scan analysis or other brain imaging techniques, allowing the detection of specific markers in clinical studies, that may then be identified as diagnostic markers.

By 'diagnostic analysis' according to the invention, it means in particular the transfer into the brain of labelled molecules for PET scan analysis or other brain imaging techniques allowing the detection of markers for the diagnostic of specific brain pathologies, such as Alzheimer (beta amyloid plaques), Parkinson (alpha-synuclein aggregates) or other early markers for these pathologies.

### Therapeutic agents for the treatment of a neurological or psychiatric disorder

In another particular embodiment, for use in therapy, the biomolecule linked to the single domain antibody directed against mGluR2 or a polypeptide comprising at least one single domain antibody directed against mGluR2 used in the present invention, is a therapeutic agent for the treatment of a neurological or psychiatric disorder.

The therapeutic agent may be directly linked to the nanobody or polypeptide used in the present invention, or linked via a linker such as the ones disclosed above.

In a particular embodiment, the therapeutic agent for the treatment of a neurological or psychiatric disorder is selected in the group consisting of peptides, nanobodies, and chemical compounds.

In particular, the nanobodies used as biomolecule linked are nanobodies distinct from nanobodies directed against mGluR2.

As examples of peptides that may be used for the treatment of a neurological or psychiatric disorder, mention may be made of natural neuropeptides. As examples of nanobodies distinct from nanobodies directed against mGluR2 that may be used for the treatment of a neurological or psychiatric disorder, mention may be made of nanobodies targeting amyloid plaques.

As examples of chemical compounds that may be used for the treatment of a neurological or psychiatric disorder, mention may be made of chemical compounds with poor brain penetration or of positive allosteric modulators of mGluR2. Various compounds have been described as mGluR2 positive allosteric modulators. WO2004/092135 (NPS & Astra Zeneca), WO2004/018386, WO2006/014918 and WO2006/015158 (Merck), WO2001/56990 (Eli Lilly) and WO2006/030032 and WO2007/104783 (Addex & Janssen Pharmaceutica) describe respectively phenyl sulfonamide, acetophenone, indanone, pyridylmethyl sulfonamide and pyridinone derivatives as mGluR2 positive allosteric modulators. None of the specifically disclosed compounds therein are structurally related to the single domain antibodies or polypeptides of the present invention. In some embodiments, the single domain antibody used in the present invention is used in combination with a compound selected from the group consisting of ADX-71149, GSK1331258, Imidazo[1,2-a]pyridines, 3-Aryl-5-phenoxymethyl-1,3-oxazolidin-2-ones, 3-(Imidazolyl methyl)-3-aza-bicyclo[3.1.0]hexan-6-yl)methyl ethers, BINA, and LY-487,379.

By 'therapeutic uses' according to the invention, it means treatment or prevention of neurological and psychiatric disorders associated with glutamate dysfunction and diseases in which the mGluR2 subtype of metabotropic receptors is involved.

In particular, the neurological disorder associated with glutamate dysfunction is selected from the group consisting of cerebral ischemia, head trauma, neurodegeneration, Alzheimer's disease, epilepsy, and pain, or or psychiatric disorder associated with glutamate dysfunction is selected from the group consisting of psychosis, schizophrenia, anxiety, depression, and substance-related disorder or addiction/drug or alcohol dependence.

In a particular and preferred embodiment, the disorder is schizophrenia.

The present invention also relates to a combination product comprising an i) isolated single domain antibody directed against mGluR2 or a polypeptide comprising at least one single domain antibody directed against mGluR2 of the invention linked to ii) a therapeutic agent for the treatment of a neurological or psychiatric disorder, in particular selected from peptides, nanobodies distinct from nanobodies directed against mGluR2, and chemical compounds, with the proviso that the peptide does not comprise a binding domain directed against a receptor on the vascular endothelium of the blood-brain barrier or a Fc portion of an immunoglobulin binding domain.

In a first embodiment, the combination product comprises an i) isolated single domain antibody directed against mGluR2 or a polypeptide comprising at least one single domain antibody directed against mGluR2 of anyone of the invention linked to ii) a peptide. Examples of linkers and peptides are disclosed above.

In a second embodiment, the combination product comprises an i) isolated single domain antibody directed against mGluR2 or a polypeptide comprising at least one single domain antibody directed against mGluR2 of anyone of the invention linked to ii) a nanobody distinct from nanobodies directed against mGluR2. Examples of linkers and nanobodies distinct from nanobodies directed against mGluR2 are disclosed above.

In a third embodiment, the combination product comprises an i) isolated single domain antibody directed against mGluR2 or a polypeptide comprising at least one single domain antibody directed against mGluR2 of anyone of the invention linked to ii) a chemical compound. Examples of linkers and chemical compounds are disclosed above.

### EXAMPLES

### Example 1: Materials and Methods

### mGlu2-targeting nanobodies constructs

Several monovalent nanobodies (IGF-1159, IGF-1160, IGF-1163, IGF-1164, IGF-1166, IGF-1168) and bivalent nanobody (IGF-1202) were used in the following examples

**Production and purification of nanobody.** For large scale nanobody production, pHEN plasmid vector with the nanobody constructs were transformed in *E. coli* BL21DE3 strain (Thermofisher). A single colony of each construct was grown into 200 mL of LB, supplemented with 100 µg.mL-1 ampicillin, 1% (wt/vol) glucose, overnight at 37°C with shaking. Then 1 L of LB supplemented with around 30 mL of the preculture and incubated until an OD600 of 0.6 - 0.7. The nanobody expression was then induced with 1 mM Isopropyl B-D-1-thiogalactopyranoside (IPTG) (final concentration) and bacteria were grown at 28°C with shaking. Bacteria were then collected by centrifugation for 10 min at 5000xg, resuspended in 10 mL per 1 L culture of ice-cold TES buffer (0.2 M Tris, 0.5 mM EDTA, 0.5 M sucrose, pH 8) and incubated for at least 1 h at 4°C on shaking platform. Twenty milliliters per liter of culture of TES/4 (TES buffer dilute 4 times in water) were then added to the solution and further incubated for at least 45 min at 4°C on a shaking platform. The periplasmic extract was recovered by collecting the supernatant after centrifugation of the suspension for 30 min at 10000xg at 4°C. The His-tagged nanobodies were then purified from the periplasmic extract by using Ni-NTA purification (Qiagen, Hilden, Germany) according to the manufacturer's instructions. Desalting was performed according to the manufacturer's instructions (disposable PD-10 desalting columns, Cytiva, US) to obtain the nanobodies in phosphate buffer solution. The endotoxin was removed according to the manufacturer's instruction (Proteus NoEndoTM S (Standard), Generon, UK).

**Binding experiments by TR-FRET. mGlu2 nanobodies.** HEK-293 cells (ATCC CRL-1573) were transfected with rat mGlu2 SNAP-tagged receptors (Maurel et al. Nature Methods 2008). 24 h after transfection, cells were labeled with 100 nM SNAP-Lumi4-Tb in Tag-Lite 1x (Cisbio, PerkinElmer) for at least 1h at 37° C, 5% CO₂. Then, they were washed three times with PBS. Cells were then detached using cell dissociation buffer (Sigma Aldrich); transferred at 5 000 cells per well in a white 384-well plate (Greiner bio-one, microplate, PS, F-bottom, small volume, Hibase, medium binding). The His-tagged nanobodies with 1 µM LY379268 (Tocris, Bio-techne, France) or 1 µM LY341495 (Tocris, Bio-techne, France) or buffer and 200 nM of anti-6His-d2 (Tocris, Bio-techne, France) were applied on labeled cells in a final volume of 20 µL and incubated overnight at room temperature. FRET signal was determined by measuring the sensitized acceptor emission (665 nm) and Tb donor emission (620 nm) using a 60 µs delay and 400 µs integration upon excitation at 337 nm on a PHERAstar FS (BMG LabTech, Ortenberg, Germany). TR-FRET (or HTRF) ratio (665 nm/620 nm ×10⁴, Cisbio patent). Affinities (pKd values) determined are reported in Table 4.

### Table 4

**Table 4 : pKd values of nanobodies for the active and inactive conformations of the indicated mGlu receptors and pEC50 values of the PAM effect on G protein coupling. n.b. : no binding, n.t. : not tested**

| | | **Binding active conformation** | Binding **inactive conformation** | **PAM effect G protein coupling** |
|---|---|---|---|---|
| | | pKd | pKd | pEC50 |
| mGlu2 | **IGF-1159** | 8.46 | *n.b.* | 6.96 |
| | **IGF-1201** | 9.34 | 8.95 | 8.25 |
| | **IGF-1202** | 9.27 | 8.87 | 8.81 |
| | **IGF-1251** | 8.99 | 8.54 | 7.65 |
| | **IGF-1252** | 8.77 | 8.49 | 7.94 |
| | **IGF-1261** | 8.51 | *n.b.* | 7.62 |
| | **IGF-1262** | 8.23 | *n.b.* | 7.20 |
| **mGlu4** | **IGF-1169** | 8.07 | *8.11* | *n.t.* |
| **mGlu7** | **IGF-1180** | 7.79 | 7.72 | *n.t.* |
| **mGlu5** | **IGF-1174** | 7.24 | *n.t.* | *n.t.* |
| | **IGF-1176** | 7.30 | *n.t.* | *n.t.* |
| | IGF-1177 | 7.24 | *n.t.* | *n.t.* |
| | **IGF-1156** | 6.90 | *n.t.* | *n.t.* |

**Binding experiments by TR-FRET. mGlu4 nanobodies.** HEK-293 cells were transfected with rat mGlu4 SNAP-tagged receptors (Doumazane et al., 2011). Cells were transferred at 100 000 cells per well into a 96-well plate (Greiner bio-one, microplate, F-bottom). 24 h after transfection, they were labeled with 100 nM SNAP-Lumi4-Tb in DMEM-GlutaMAX for 1h30 at 37°C, 5% CO₂, washed three times with Tag-Lite 1x (Cisbio, PerkinElmer). Labeled cells were pre-incubated with 10 µM L-AP4 (Tocris, Bio-techne, France)or 100 µM LY341495 (Tocris, Bio-techne, France) or buffer for at least 30min. The His-tagged nanobodies and 200 nM of anti-c-myc-d2 (Perkin-Elmer-CisBio, France) were added in a final volume of 90 µL and incubated overnight at room temperature. FRET signal was determined by measuring the sensitized acceptor emission (665 nm) and Tb donor emission (620 nm) using a 60 µs delay and 400 µs integration upon excitation at 337 nm on a PHERAstar FS (BMG LabTech, Ortenberg, Germany). TR-FRET (or HTRF^{®}) ratio (665 nm/620 nm ×10⁴, Cisbio patent). Affinities (pKd values) determined are reported in Table 4.

Binding experiments by TR-FRET. mGlu5 nanobodies. HEK-293 cells were transfected with rat mGlu5 SNAP-tagged receptors (Doumazane et al., 2011). 24 h after transfection, cells were labeled with 100 nM SNAP-Lumi4-Tb in Tag-Lite 1x (Cisbio, PerkinElmer) for at least 1h at 37° C, 5% CO₂. Then, they were washed three times with PBS. Cells were then detached using cell dissociation buffer (Sigma Aldrich); transferred at 10 000 cells per well in a white 384-well plate (Greiner bio-one, microplate, PS, F-bottom, small volume, Hibase, medium binding). The His-tagged nanobodies with 10µM quisqualate or 100 µM LY341495 or buffer and 200 nM of anti-6His-d2 (Perkin-Elmer-CisBio, France) were applied on labeled cells in a final volume of 20 µL and incubated overnight at room temperature. FRET signal was determined by measuring the sensitized acceptor emission (665 nm) and Tb donor emission (620 nm) using a 60 µs delay and 400 µs integration upon excitation at 337 nm on a PHERAstar FS (BMG LabTech, Ortenberg, Germany). TR-FRET (or HTRF^{®}) ratio (665 nm/620 nm ×10⁴, Cisbio patent). Affinities (pKd values) determined are reported in Table 4.

**Binding experiments by TR-FRET. mGlu7 nanobodies.** HEK-293 cells were transfected with rat mGlu7 SNAP-tagged receptors (Doumazane et al., 2011). Cells were transferred at 100 000 cells per well into a 96-well plate (Greiner bio-one, microplate, F-bottom). 24 h after transfection, they were labeled with 300 nM SNAP-Lumi4-Tb in DMEM-GlutaMAX for 1h30 at 37°C, 5% CO₂, washed three times with Tag-Lite 1x (Cisbio, PerkinElmer). Labeled cells were pre-incubated with 10 µM LSP4-2022 (Selvam et al., 2018) or 100 µM LY341495 or buffer for at least 30 min. The His-tagged nanobodies and 200 nM of anti-c-myc-d2 (Perkin-Elmer, CisBio, France) were added in a final volume of 60 µL and incubated overnight at room temperature. FRET signal was determined by measuring the sensitized acceptor emission (665 nm) and Tb donor emission (620 nm) using a 60 µs delay and 400 µs integration upon excitation at 337 nm on a PHERAstar FS (BMG LabTech, Ortenberg, Germany). TR-FRET (or HTRF^{®}) ratio (665 nm/620 nm ×10⁴, Cisbio patent). Affinities (pKd values) determined are reported in **Table 4.**

**Immunohistochemistry on brain slices.** Experiments were conducted on adult male C57BL/6J mice purchased from Janvier Labs. Mice were injected intraperitoneally with 10 mg/kg of nanobody or with an equivalent volume of PBS (100-150 µl) as a negative control. 24-31 h post-injection, mice were anesthetized and sacrificed by an intracardiac perfusion of 4% PFA. Brains were extracted and fixed with a 4% PFA solution, overnight at 4°C. Brains were then rinsed in PBS and cryoprotected with a 30% sucrose solution for 4 days at 4°C. Brains were included in Tissue-Tek^{®} optimum cutting temperature (O.C.T.) compound (Sakura Finetek, France) and conserved at -80°C. Brains included in Tissue-Tek^{®} O.C.T. compound were mounted on a cryostat (Leica, Nanterre, France) and 16 µm sagittal sections were performed directly mounted on SuperFrost Plus glass slides (Microm) and kept at -20°C, until use. Three glass slides per animal, each containing 5 brain tissue sections, with 80 µm spacemen between each section, were rinsed with TBS and incubated 1 h at room temperature with a blocking solution (5% normal goat serum (Invitrogen, Rockford, US) - 0.1% Triton X100 - TBS). Sections were then incubated with a monoclonal rabbit anti-6xHis (1:500, Life Technologies) 2 h at room temperature, washed in TBS and incubated with a goat anti-rabbit biotinylated (ABC kit ,1:100, Vector Laboratories). Labeling was performed using 3-3'-diaminobenzidine chromogen solution according to the instructions provided with the Strept ABC complex kit (Vector Laboratories). Sections were washed with distilled water and mounted using Fluoroshield with DAPI. Images were taken using a slide scanner AxioScan z1 microscope by performing full-section mosaics with a 20x enlargement.

**Statistical analysis.** Quantitative data are shown as means, with error bars indicating the standard error of the mean (SEM). One-way ANOVA and non-parametric Kruskal-Wallis tests were used to compare more than two groups. All values for which p<0.05 were considered significant. Statistical analyses were performed with GraphPad Prism 7 et 9 (GraphPad software Inc, San Diego, CA, USA).

**Ethics.** Care of the animals were performed according to the Directive 2010/63/EU of the European Parliament, which had been approved by the Ministry of Agriculture, France. The project was submitted to the French Ethic Committee CEEA (Comité d'Ethique en Expérimentation Animale) and obtained the authorization APAFIS #31981-2021060423426200 v3. All experiments were performed in accordance with relevant named guidelines and regulations.

**Nanobody radiolabeling.** IGF-1202 was radiolabeled using bacterial transglutaminase and a systetic peptide rag, CBz-Gln-Gly-Lys-OH. *Synthesis of the peptide tag: CBz- Gln-Gly-Lys-OH.* The solid phase peptide synthesis was carried out manually on a microwave synthesizer (CEM corporation) following a standard Fmoc-based synthesis protocol. Starting from the Fmoc-Lys(Boc)-Wang resin loaded at 0.32 mmol/g (Novabiochem) the synthesis was performed on a 0.015 mmol/reactor scale. Protected amino acids Fmoc-Gly-OH and CBz-Gln-OH (5 equiv.) were coupled using HATU (4.5 equiv.) and iPr2EtN (10 equiv.) in DMF for 20 minutes at 60°C. Fmoc group was deprotected by three successive treatments with 20% piperidine in DMF for 3 minutes. The peptide was then deprotected and cleaved from the resin by a treatment with trifluoroacetic acid/H2O/triisopropylsilane (95/2.5/2.5) for 2 h at room temperature. The peptide was then precipitated by dilution into an ice-cold diethyl ether solution, recovered by centrifugation and washed twice with diethyl ether.

*Radiolabeling of the peptide tag: CBz-Gln-Gly-Lys([3H]Pr)- OH.* To a solution of the CBz-protected peptide (87 nmol, 1 equiv.) in DMF (50 µL) at room temperature was added iPr2EtN (870 nmol, 10 equiv.) and [3H]N- (propionyloxy)succinimide (87 nmol, 1 equiv.). The mixture was stirred at room temperature for 2 hours. The labeled product was purified by HPLC equipped with a Luna Omega C18 (150 × 4.6 mm; 3 µm) column (Phenomenex) with a gradient of 0-100% buffer B in buffer A over 20 minutes with a flow rate of 1 mL/min (buffer A: 0.1% formic acid in H₂O, buffer B: 0.1% formic acid in acetonitrile).

*Conjugation of the radiolabeled tag on IGF-120.* The radiolabeling of IGF-1202 through lysine conjugation was carried out in PBS buffer at pH=7.4 using 1U/20 nmol of bacterial transglutaminase (Zedira), 2 equivalents of the radiolabeled peptide tag and at a concentration of 80 µM of IGF-1202 protein. The mixture was then incubated for 2 h at room temperature. After incubation, the labeled protein was purified on Protino Ni-NTA resin (Macherey-Nagel) and then buffer was exchanged to NaCl 0.9% using Amicon centrifugal filter (Merck) for *in vivo* experiments. Part of the labeled IGF-1202 was digested by trypsin to identify Lys121 as the conjugation site by MALDI-TOF (Applied Biosystems) analysis.

***In vivo* cerebral quantification and localization of the tritiated nanobody.** Experiments were conducted on adult female C57BL/6J mice (21.5 ± 0.3 g) purchased from Janvier Labs. Mice were injected intraperitoneally with 10 mg/kg of [³H]- IGF-1202 in 50 µL. Blood and urine samples were collected 4 h after intraperitoneal injection. Animals were euthanized 4 h, 24 h or 7 days after administration, perfused with NaCl 0.9% (10 mL/mouse, 200 mL/h) for brain collection and freezing. Brains were used for either tissue quantification, or cerebral localization of β-radioactivity. Quantification of β-radioactivity contained in total brain homogenate was performed using a liquid scintillation analyzer (Tri-Carb 2100TR, Packard BioScience Company, USA). Brain tissue sections (20 µm thick) were prepared on a microtome to perform imaging and observe cerebral localization by digital autoradiography with a β-IMAGER (Biospace Lab, France). *Statistical analysis.* Quantitative data are shown as means, with error bars indicating the standard error of the mean (SEM). Non-parametric Kruskal- Wallis test was used to compare more than two groups. All values for which p<0.05 were considered significant. Statistical analyses were performed with GraphPad Prism 8 (GraphPad software Inc, San Diego, CA, USA).

*Ethics.* Care of the animals and surgical procedures were performed according to the Directive 2010/63/EU of the European Parliament, which had been approved by the Ministry of Agriculture, France. The project was submitted to the French Ethic Committee CEEA (Comité d'Ethique en Expérimentation Animale) and obtained the authorization APAFIS#4111-2016021613253432 v5. All experiments were performed in accordance with relevant named guidelines and regulations.

### REFERENCES

Bickel, U, Yoshikawa, T, Pardridge WN et al. Delivery of peptides and proteins through the blood-brain barrier. Adv Drug Deliv Rev 46(1-3), 247-279 (2001).

Choi JJ et al. Noninvasive and transient bloodbrain barrier opening in the hippocampus of Alzheimer's double transgenic mice using focused ultrasound. Ultrasound Imaging 30:189-200 (2008).

Doumazane, E. et al. A new approach to analyze cell surface protein complexes reveals specific heterodimeric metabotropic glutamate receptors. FASEB J 25, 66-77 (2011).

Gerbier R, Alvear-Perez R, Margathe JF, Flahault A, Couvineau P, Gao J, De Mota N, Dabire H, Li B, Ceraudo E, Hus-Citharel A, Esteoulle L, Bisoo C, Hibert M, Berdeaux A, Iturrioz X, Bonnet D, Llorens-Cortes C. Development of original metabolically stable apelin-17 analogs with diuretic and cardiovascular effects. FASEB J 31 (2):687-700 (2017).

Hutt M, Färber-Schwarz A, Unverdorben F, Richter F, Kontermann RE. Plasma half-life extension of small recombinant antibodies by fusion to immunoglobulin-binding domains. J Biol Chem 287 (3), 4462-4469 (2012).

Karlin S, Altschul SF. Methods for assessing the statistical significance of molecular sequence features by using general scoring schemes. Proc Natl Acad Sci U S A 87(6):2264-8 (1990).

Maurel et al., 2008. Cell-surface protein-protein interaction analysis with time-resolved FRET and snap-tag technologies: application to GPCR oligomerization. Nature Methods volume 5, pages561-567 (2008).

Selvam et al., 2018. Increased Potency and Selectivity for Group III Metabotropic Glutamate Receptor Agonists Binding at Dual sites. J Med Chem 61, 5, 1969-1989 (2018).

Stork R, Campigna E, Robert B, Müller D, Kontermann RE. Biodistribution of a bispecific single-chain diabody and its half-life extended derivatives. J Biol Chem 18;284(38):25612-9 (2009).

Walker A, Dunlevy G, Rycroft D, Topley P, Holt LJ, Herbert T, Davies M, Cook F, Holmes S, Jespers L, Herring C. Anti-serum albumin domain antibodies in the development of highly potent, efficacious and long-acting interferon. Protein Eng Des Sel 23 (4):271-8 (2010).

Wu Sk et al. Pulsed-wave low-dose ultrasound hyperthermia selectively enhances nanodrug delivery and improves antitumor efficacy for brain metastasis of breast cancer. Ultrason Sonochem 36:198-205 (2017).

## Claims

1. An isolated single domain antibody directed against mGluR2 or a polypeptide comprising at least one single domain antibody directed against mGluR2 for use as a biomolecule transporter crossing the blood-brain barrier (BBB).

2. The isolated single domain antibody directed against mGluR2 or the polypeptide comprising at least one single domain antibody directed against mGluR2 for use according to claim 1 wherein the single domain antibody directed against mGluR2 comprises :
a CDR1 having a sequence set forth as SEQ ID NO:1, a CDR2 having a sequence set forth as SEQ ID NO:2 and a CDR3 having a sequence set forth as SEQ ID NO:3; or
a CDR1 having a sequence set forth as SEQ ID NO 4, a CDR2 having a sequence set forth as SEQ ID NO:5 and a CDR3 having a sequence set forth as SEQ ID NO:6 ; or
a CDR1 having a sequence set forth as SEQ ID NO:7 , a CDR2 having a sequence set forth as SEQ ID NO:8 and a CDR3 having a sequence set forth as SEQ ID NO:9; or
a CDR1 having a sequence set forth as SEQ ID NO:10 a CDR2 having a sequence set forth as SEQ ID NO:11 and a CDR3 having a sequence set forth as SEQ ID NO:12 or
a CDR1 having a sequence set forth as SEQ ID NO:13 a CDR2 having a sequence set forth as SEQ ID NO:14 and a CDR3 having a sequence set forth as SEQ ID NO:15; or
a CDR1 having a sequence set forth as SEQ ID NO:16, a CDR2 having a sequence set forth as SEQ ID NO:17 and a CDR3 having a sequence set forth as SEQ ID NO:18.

3. The isolated single domain antibody directed against mGluR2 or the polypeptide comprising at least one single domain antibody directed against mGluR2 of claim 2 for use according to claim 1, wherein the single domain antibody directed against mGluR2 has at least 85% identity with anyone of the sequences set forth as SEQ ID NO:19 to SEQ ID NO: 21, preferably at least 90% identity, more preferably at least 95% identity, and even 100% identity with anyone of the sequences set forth as SEQ ID NO:19 to SEQ ID NO: 21.

4. The isolated single domain antibody directed against mGluR2 or the polypeptide comprising at least one single domain antibody directed against mGluR2 of claim 3, wherein the single domain antibody directed against mGluR2 has the sequence set forth as SEQ ID NO:19.

5. The isolated single domain antibody directed against mGluR2 or the polypeptide comprising at least one single domain antibody directed against mGluR2 of claim 3, wherein the single domain antibody directed against mGluR2 has the sequence set forth as SEQ ID NO:21.

6. The polypeptide for use according to claim 1, which is a biparatopic polypeptide comprising
(i) one single domain antibody having
a CDR1 having a sequence set forth as SEQ ID NO:1, a CDR2 having a sequence set forth as SEQ ID NO:2 and a CDR3 having a sequence set forth as SEQ ID NO:3; or
a CDR1 having a sequence set forth as SEQ ID NO:4, a CDR2 having a sequence set forth as SEQ ID NO:5 and a CDR3 having a sequence set forth as SEQ ID NO:6; or
a CDR1 having a sequence set forth as SEQ ID NO:7, a CDR2 having a sequence set forth as SEQ ID NO:8 and a CDR3 having a sequence set forth as SEQ ID NO:9; and
(ii) another single domain antibody having
a CDR1 having a sequence set forth as SEQ ID NO:10, a CDR2 having a sequence set forth as SEQ ID NO:11 and a CDR3 having a sequence set forth as SEQ ID NO:12; or
a CDR1 having a sequence set forth as SEQ ID NO:13, a CDR2 having a sequence set forth as SEQ ID NO:14 and a CDR3 having a sequence set forth as SEQ ID NO:15; or
a CDR1 having a sequence set forth as SEQ ID NO:16, a CDR2 having a sequence set forth as SEQ ID NO:17 and a CDR3 having a sequence set forth as SEQ ID NO:18.

7. The biparatopic polypeptide of claim 6 for use according to claim 1, comprising (i) one single domain antibody having a CDR1 having a sequence set forth as SEQ ID NO:1, a CDR2 having a sequence set foth as SEQ ID NO:2 and a CDR3 having a sequence set forth as SEQ ID NO:3; and (ii) another single domain antibody having a CDR1 having a sequence set forth as SEQ ID NO:16, a CDR2 having a sequence set foth as SEQ ID NO:17 and a CDR3 having a sequence set forth as SEQ ID NO:18.

8. The biparatopic polypeptide of claim 7 for use according to claim 1, comprising one single domain antibody having at least 85% identity, preferably at least 90% identity, more preferably at least 95% identity, and even 100% identity with the sequence set forth as SEQ ID NO:19, and another single domain antibody having at least 85% identity, preferably at least 90% identity, more preferably at least 95% identity, and even 100% identity with the sequence set forth as SEQ ID NO:24.

9. The isolated single domain antibody directed against mGluR2 or the polypeptide comprising at least one single domain antibody directed against mGluR2 of anyone of claims 1 to 8 for use as a biomolecule transporter crossing the blood-brain barrier (BBB), wherein the biomolecule is a detectable label.

10. The isolated single domain antibody directed against mGluR2 or the polypeptide comprising at least one single domain antibody directed against mGluR2 of anyone of claims 1 to 8 for use according to claim 9, wherein the detectable label is selected from the group consisting of radioisotope labels, fluorescent labels, chemiluminescent labels, enzyme labels, and bioluminescent labels.

11. The isolated single domain antibody directed against mGluR2 or the polypeptide comprising at least one single domain antibody directed against mGluR2 of anyone of claims 1 to 8 for use according to claim 9 or 10, for use in biological analysis and/or diagnostic analysis.

12. The isolated single domain antibody directed against mGluR2 or the polypeptide comprising at least one single domain antibody directed against mGluR2 of anyone of claims 1 to 8 for use as a biomolecule transporter crossing the blood-brain barrier (BBB), wherein the biolomecule is a therapeutic agent for the treatment of a neurological or psychiatric disorder.

13. The isolated single domain antibody directed against mGluR2 or the polypeptide comprising at least one single domain antibody directed against mGluR2 of anyone of claims 1 to 8 for use according to claim 12, wherein the therapeutic agent for the treatment of a neurological or psychiatric disorder is selected in the group consisting of peptides, nanobodies, and chemical compounds.

14. The isolated single domain antibody directed against mGluR2 or the polypeptide comprising at least one single domain antibody directed against mGluR2 of anyone of claims 1 to 8 for use according to claim 13, wherein neurological or psychiatric disorder is selected from the group consisting of cerebral ischemia, head trauma, neurodegeneration, Alzheimer's disease, epilepsy, and pain, or or psychiatric disorder associated with glutamate dysfunction is selected from the group consisting of psychosis, schizophrenia, anxiety, depression, and substance-related disorder or addiction/drug or alcohol dependence.

15. A combination product comprising an i) isolated single domain antibody directed against mGluR2 or a polypeptide comprising at least one single domain antibody directed against mGluR2 of anyone of claims 1 to 8 linked to ii) a therapeutic agent for the treatment of a neurological or psychiatric disorder, in particular selected from peptides, nanobodies distinct from nanobodies directed against mGluR2, and chemical compounds, with the proviso that the peptide does not comprise a binding domain directed against a receptor on the vascular endothelium of the blood-brain barrier or a Fc portion of an immunoglobulin binding domain.
